(19) Europäisches Patentamt
European Patent Office
Office européen des brevets

(11) Numéro de publication: **0 518 769 B1**

# (12) FASCICULE DE BREVET EUROPEEN

(45) Date de publication de fascicule du brevet: **07.12.94**

(51) Int. Cl.[5]: **C07C 233/00**, C07C 219/00, A61K 31/00

(21) Numéro de dépôt: **92401620.7**

(22) Date de dépôt: **12.06.92**

Le dossier contient des informations techniques présentées postérieurement au dépôt de la demande et ne figurant pas dans le présent fascicule.

(54) **Nouveaux dérivés de benzoate d'éthanolamine, leur procédé de préparation et les compositions pharmaceutiques les renfermant.**

(30) Priorité: **14.06.91 FR 9107289**

(43) Date de publication de la demande:
**16.12.92 Bulletin 92/51**

(45) Mention de la délivrance du brevet:
**07.12.94 Bulletin 94/49**

(84) Etats contractants désignés:
**AT BE CH DE DK ES FR GB GR IT LI LU NL PT SE**

(56) Documents cités:
**EP-A- 0 061 907**
**FR-A- 1 570 822**
**FR-A- 2 356 631**
**GB-A- 1 112 526**
**US-A- 4 237 165**

(73) Titulaire: **ADIR ET COMPAGNIE**
**1 rue Carle Hébert**
**F-92415 Courbevoie Cédex (FR)**

(72) Inventeur: **Wierzbicki, Michel**
**8 Résidence du Chemin Pavé**
**F-78620 L'Etang la Ville (FR)**
Inventeur: **Hugon, Pierre**
**13 rue Eugène Sue**
**F-92500 Rueil Malmaison (FR)**
Inventeur: **Duhault, Jacques**
**14bis rue Paul Demange**
**F-78290 Croissy sur Seine (FR)**
Inventeur: **Lacour,Françoise**
**44 avenue du Château**
**F-94300 Vincennes (FR)**
Inventeur: **Boulanger, Michelle**
**"Le val Cort" 71, avenue Auguste Renoir**
**F-78160 Marly le Roi (FR)**

(74) Mandataire: **Reverbori, Marcelle**
**ADIR**
**1, rue Carle Hébert**
**F-92415 Courbevoie Cédex (FR)**

EP 0 518 769 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

| | |
|---|---|
| EUROPEAN JOURNAL OF PHARMACOLOGY vol. 141, no. 2, 1987, pages 243 - 251; JEAN-CLAUDE HENOUIN ET AL.: 'STIMULATION OF INSULIN RELEASE BY BENZOIC ACID DERIVATIVES RELATED TO THE NON-SULPHONYLUREA MOIETY OF GLIBENCLAMIDE: STRUCTURAL REOUIREMENTS AND CELLULAR MECHANISMS' | |

## Description

La présente invention a pour objet de nouveaux dérivés de benzoate d'éthanolamine, leur procédé de préparation et les compositions pharmaceutiques les renfermant.

Elle concerne particulièrement les dérivés de benzoate d'éthanolamine de formule générale I :

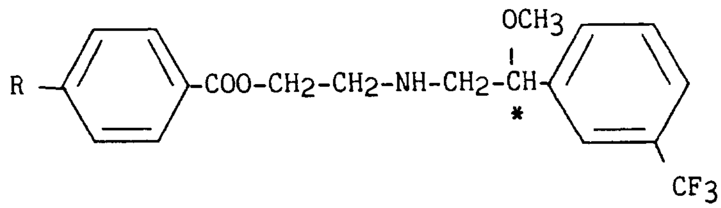

(I)

dans laquelle :

R représente :

- un atome d'hydrogène, ou
- un groupe de formule :

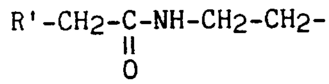

dans laquelle R' représente :

**a)** soit un radical de formule :

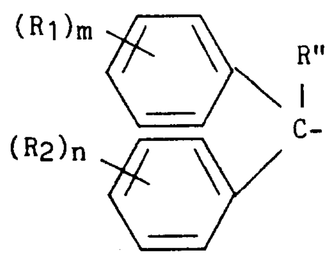

dans laquelle :

R'' représente un atome d'hydrogène ou un radical alkyle contenant de 1 à 5 atomes de carbone en chaine droite ou ramifiée,

$R_1$ et $R_2$, identiques ou différents, représentent chacun un atome d'hydrogène ou un radical alkyle ou alkoxy ayant chacun de 1 à 5 atomes de carbone en chaine droite ou ramifiée, et m et n identiques ou différents représentent chacun 1, 2 ou 3 ;

**b)** soit un radical fluorényle de formule :

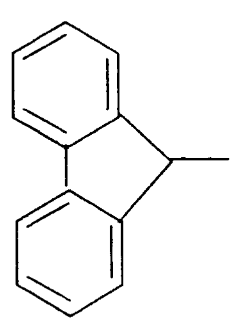

sous forme de composé racémique ou d'énantiomères.

L'état antérieur de la technique est illustré notamment :

- par les brevets français 1.517.587 et 6564 M qui ont respectivement pour objet :
  - les composés de formule :

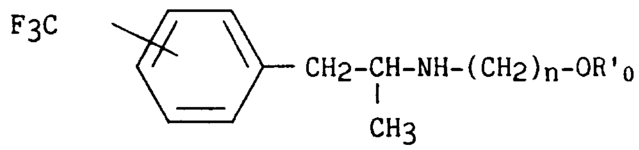

dans laquelle :

- n prend, entre autres, la valeur 2, et
  - R'$_0$ représente un atome d'hydrogène ou un groupe COR"$_0$ R"$_0$ étant, entre autres, un radical phényle éventuellement substitué, et
  - l'utilisation des composés A à titre de médicaments dans le traitement notamment de l'obésité, de la douleur et de l'épilepsie ; et
- par le US-A-4,237,165 qui concerne les compositions pharmaceutiques renfermant soit le 1-(3-trifluorométhy]phényl)-2-(β-hydroxyéthyl) amino-propane soit le 1-(3-trifluorométhylphényl)-2-(β-benzoyloxyéthyl)aminopropane ou Benfluorex (DCI), utilisables dans le traitement des troubles du métabolisme.
- par le FR-A-1 570 822 qui concerne à titre de produits chimiques nouveaux des dérivés de l'acide sulfamyl benzoïque, et enfin
- par le GB-A-1, 112, 526 qui a pour objet des alkoxy trifluorométhylphénalkylamines ayant une activité antiémétique et sédative et antagoniste de la nicotine.

Des modifications importantes de structure ont conduit aux dérivés de formule I de la présente invention, lesquels régularisent le métabolisme des glucides et des lipides, et s'opposent à l'oxydation des LDL, tout en étant sans effet sur le taux de sérotonine cérébrale, ce qui n'est pas le cas des composés de l'état de la technique ci-dessus cités qui eux sont inactifs sur l'oxydation des LDL et modifient le taux de sérotonine cérébrale comme le prouve l'étude pharmacologique décrite dans l'exemple 10.

La présente invention a également pour objet le procédé de préparation des composés de formule générale I caractérisé en ce que :

- l'on transforme l'acide de formule générale II :

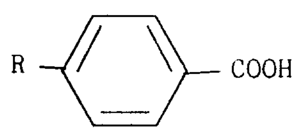

(II)

dans laquelle R a la signification précédemment définie, en un sel de formule générale II' :

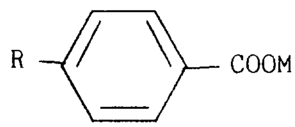

(II')

dans laquelle R a la signification précédemment définie et M est un métal alcalin ou alcalino-terreux ;

- l'on fait réagir ce dernier avec un composé halogéné de formule générale III :

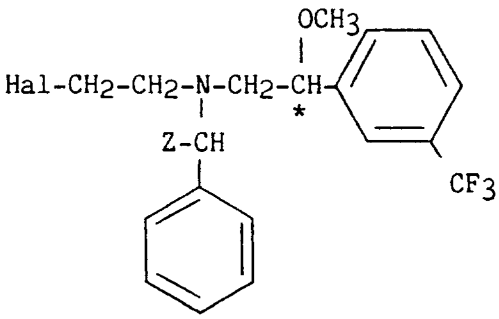

(III)

dans laquelle :

- Z représente un atome d'hydrogène ou un radical méthyle, et
- Hal représente un atome d'halogène tel qu'un atome de chlore, de brome ou d'iode ;

et l'on débenzyle catalytiquement le composé ainsi obtenu de formule générale IV :

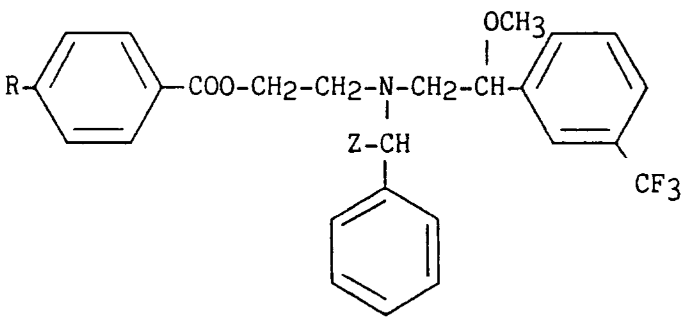

(IV)

dans laquelle R et Z ont les significations précédemment définies.

Les composés de formule générale III dans laquelle Z représente un atome d'hydrogène conduisent à des composés IV racémiques.

Les composés de formule générale III dans laquelle Z représente un radical méthyle conduisent soit à des composés IV racémiques, soit à des énantiomères lévogyres ou dextrogyres selon que le groupement $\alpha$-méthylbenzyle lié à l'atome d'azote est RS, S ou R respectivement.

Certaines matières premières de formule générale II :

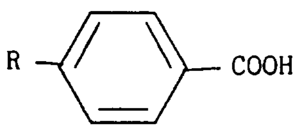

(II)

sont connues.

C'est le cas des acides de formule générale II dans laquelle R représente :

- soit un atome d'hydrogène (dans ce cas le composé II est l'acide benzoïque qui est un produit du commerce)

- soit un groupe :

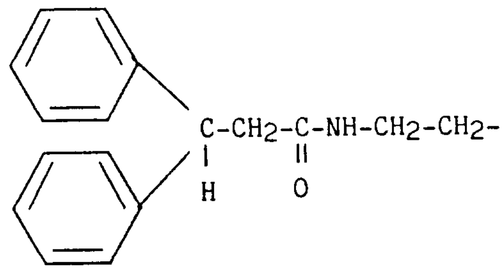

cf : European Journal of Pharmacology (1987), 141-2, 243-251 ;

- soit le radical fluorén-9-yl acétylaminoéthyl-, cf : brevet US 4.136.197.

Dans les autres cas, c'est à dire lorsque R représente un groupe :

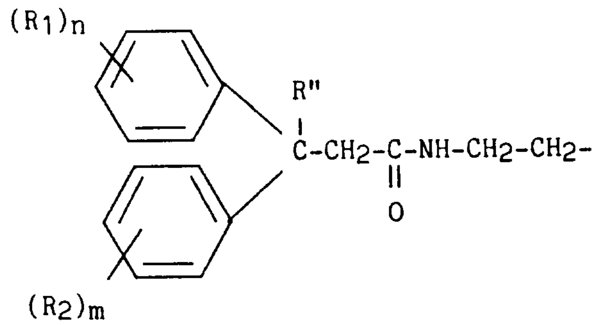

dans laquelle : $R_1$, $R_2$, R'', n et m sont tels que précédemment définis mais de plus $R_1$, $R_2$ et R'' ne représentent jamais simultanément un atome d'hydrogène, les composés II correspondants sont des produits nouveaux qui font, à ce titre, partie de la présente invention.

Sont donc inclus dans la présente invention, à titre de matières premières nouvelles utilisables pour la synthèse des composés de formule générale I -eux-mêmes utilisables comme médicaments- les composés II répondant plus spécifiquement à la formule générale IIa :

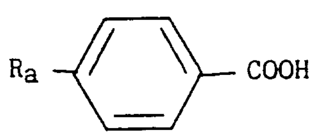

(IIa)

dans laquelle $R_a$ représente un groupe de formule générale A :

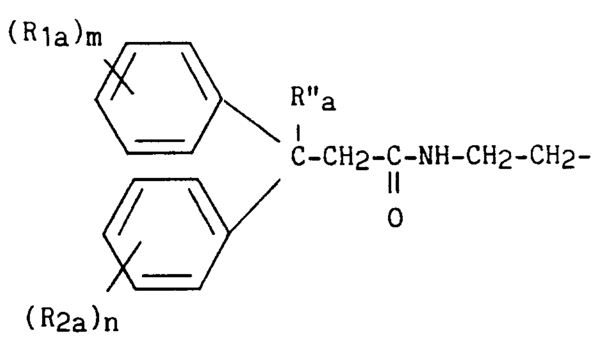

(A)

dans laquelle :

- m et n ont les significations précédemment définies,

- R"$_a$ représente un atome d'hydrogène ou un radical alkyle contenant de 1 à 5 atomes de carbone en chaine droite ou ramifiée,
- $R_{1a}$ et $R_{2a}$, identiques ou différents, représentent chacun un atome d'hydrogène ou un radical alkyle ou alkoxy ayant chacun de 1 à 5 atomes de carbone en chaine droite ou ramifiée, à condition toutefois que $R_{1a}$, $R_{2a}$ et R"a ne représentent jamais simultanément un atome d'hydrogène.

Les composés II qui sont nouveaux ont été préparés en transformant les acides de formule générale V :

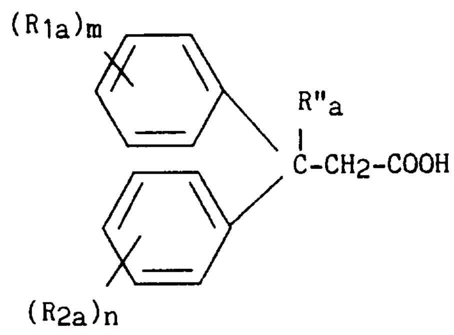

(V)

dans laquelle : R"$_a$, $R_{1a}$, $R_{2a}$, m et n ont les significations précédemment définies, en chlorure d'acide ou en anhydride mixte, respectivement de formule générale $V_a$ ou $V_b$ :

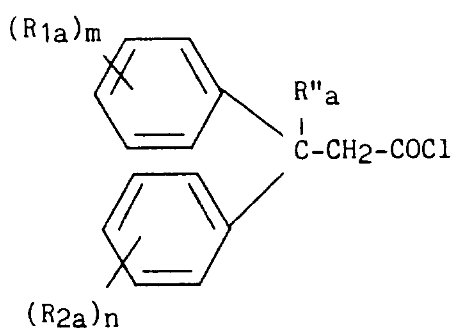

(Va)

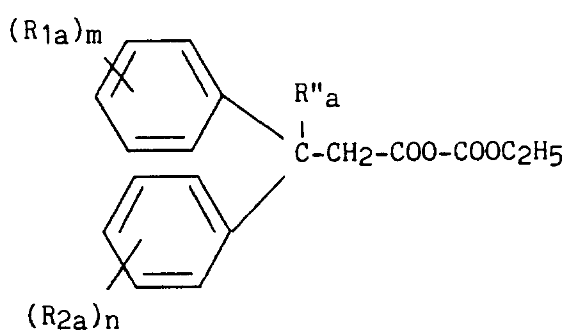

(Vb)

lesquels sont condensés

**a)** soit sur un amino acide de formule VIa :

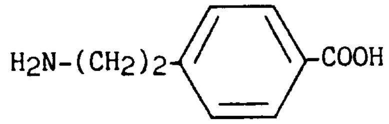

(VIa)

pour obtenir directement un composé de formule IIa, formule que l'on peut écrire plus précisément comme suit :

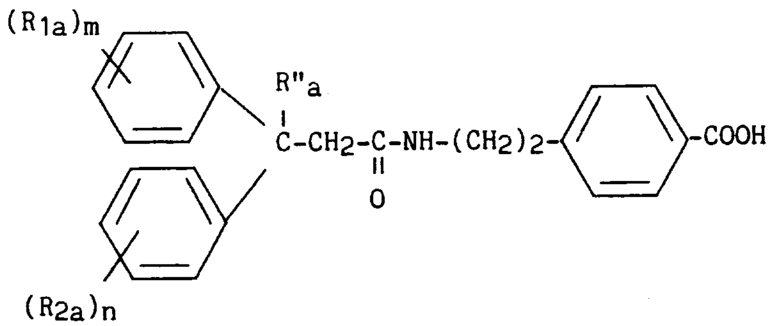

(IIa)

dans laquelle R"a, $R_{1a}$, $R_{2a}$, m et n ont les significations précédemment définies.

**b)** soit sur un amino ester de formule VIb :

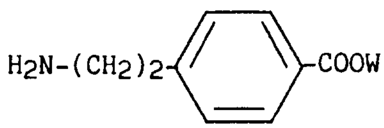

(VIb)

dans laquelle W est un radical alkyle ayant de 1 à 5 atomes de carbone en chaine droite ou ramifiée pour obtenir un composé de formule générale II'a :

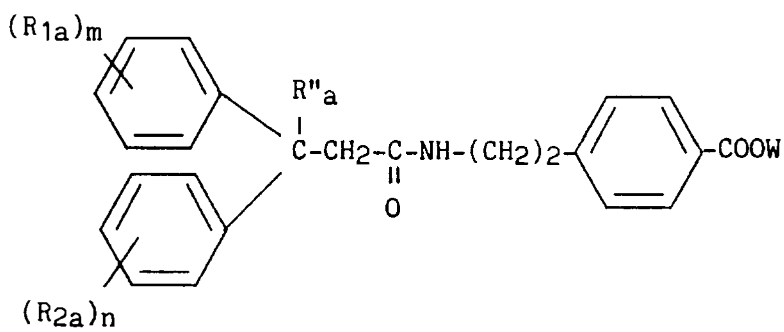

(II'a)

dans laquelle R"a, $R_{1a}$, $R_{2a}$, m, n et W ont les significations précédemment définies, lequel est hydrolysé en acide de formule IIa.

Dans le cas ou R"a représente un atome d'hydrogène, les acides V correspondants ont eux-mêmes été obtenus par réaction de HORNER entre la cétone de formule B :

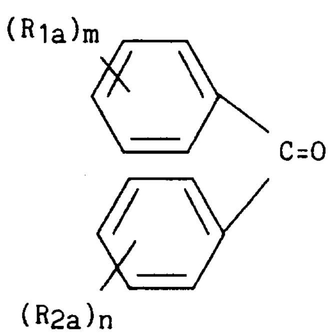

(B)

et le phosphonoacétate d'éthyle $[(C_2H_5O)_2OP-CH_2-COOC_2H_5]$ réaction suivie d'une hydrogénation catalytique du composé ainsi formé de formule C :

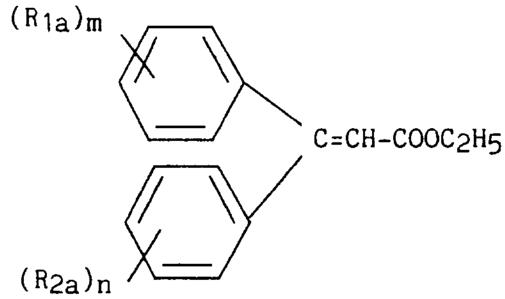

(C)

pour donner l'ester de formule D :

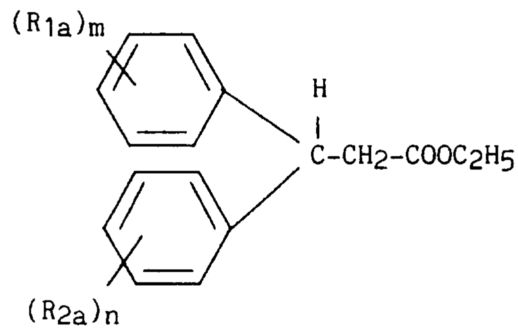

(D)

lequel est ensuite hydrolysé en acide correspondant de formule E :

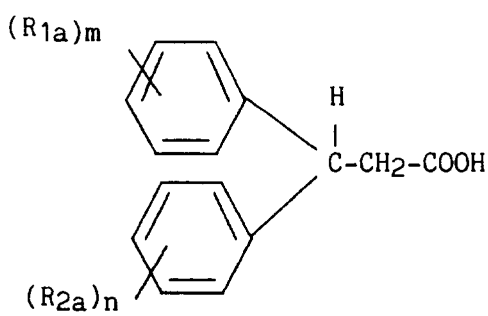

(E)

($R_{1a}$, $R_{2a}$, m et n ayant, dans toutes ces formules, les définitions précédemment définies), c'est à dire en acide V dans le cas où R'' représente un atome d'hydrogène.

Dans le cas où $R''_a$ représente un radical alkyle contenant de 1 à 5 atomes de carbone en chaine droite ou ramifiée, les acides V correspondants ont eux-mêmes été obtenus par condensation de COPE entre la cétone de formule B' :

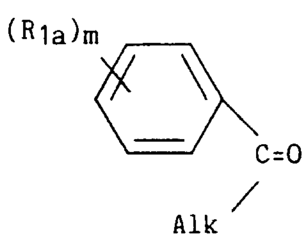

(B')

(dans laquelle $R_{1a}$ et m sont tels que précédemment définis et Alk représente un radical alkyle contenant de 1 à 5 atomes de carbone en chaine droite ou ramifiée)

et le cyanoacétate d'éthyle ($NC-CH_2-COOC_2H_5$) dans le toluène, en présence d'acide acétique et d'acétate d'ammonium pour donner le dérivé éthylénique de formule F :

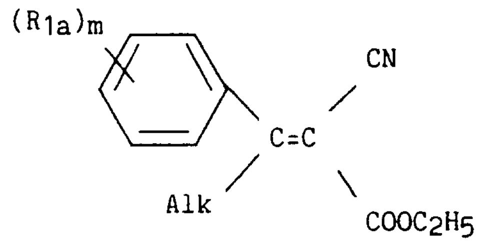

(F)

lequel est alors soumis à l'action d'un composé organomagnésien de formule G :

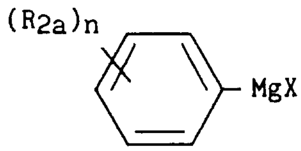

(G)

($R_{2a}$ et n étant tels que précédemment définis et X étant un atome d'halogène) pour donner le composé de formule H :

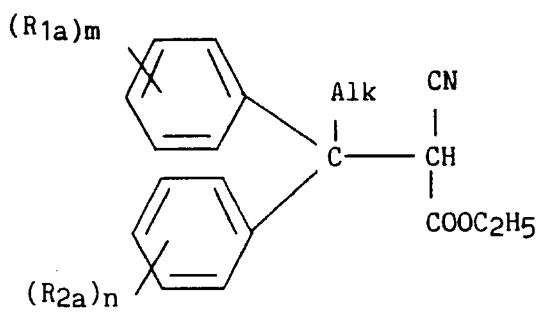

(H)

(dans laquelle $R_{1a}$, $R_{2a}$, m, n et Alk sont tels que précédemment définis) lequel composé H est hydrolysé en acide de formule J :

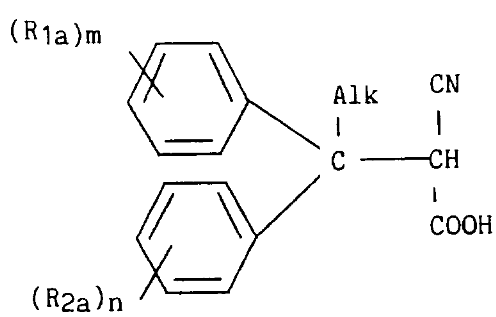

(J)

qui est décarboxylé pour donner le nitrile de formule K :

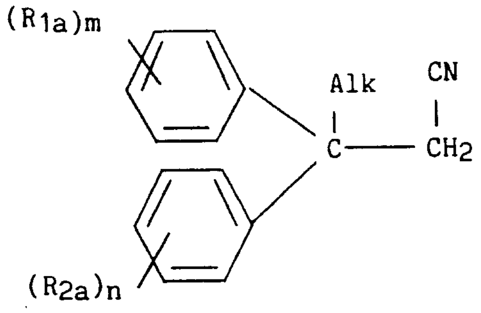

(K)

lequel est à son tour hydrolysé en acide attendu de formule L :

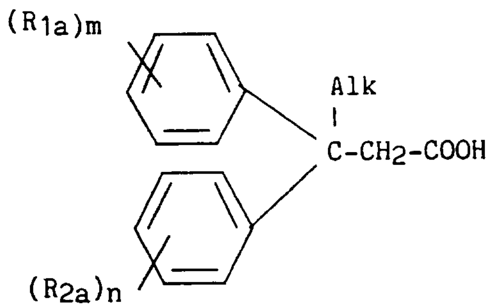

(L)

(dans laquelle $R_{1a}$, $R_{2a}$, m, n et Alk sont tels que précédemment définis) ; c'est à dire en acide de formule V dans le cas où $R''_a$ représente un radical alkyle contenant de 1 à 5 atomes de carbone en chaine droite ou ramifiée.

Les matières premières de formule générale III :

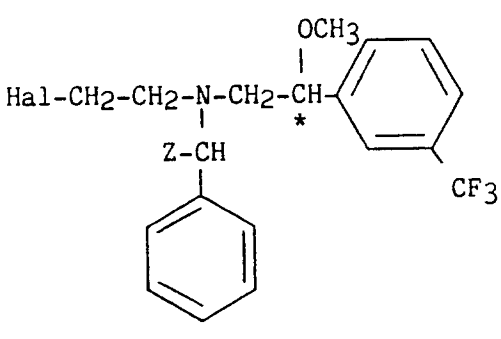

(III)

dans laquelle Hal et Z sont tels que précédemment définis, ont été obtenues selon le processus réactionnel suivant :

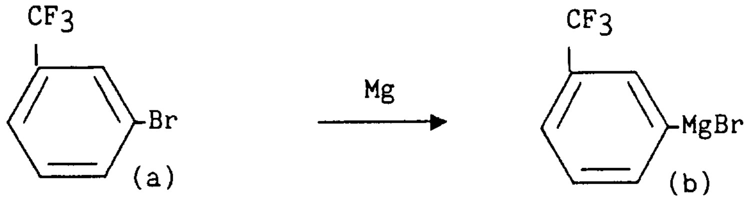


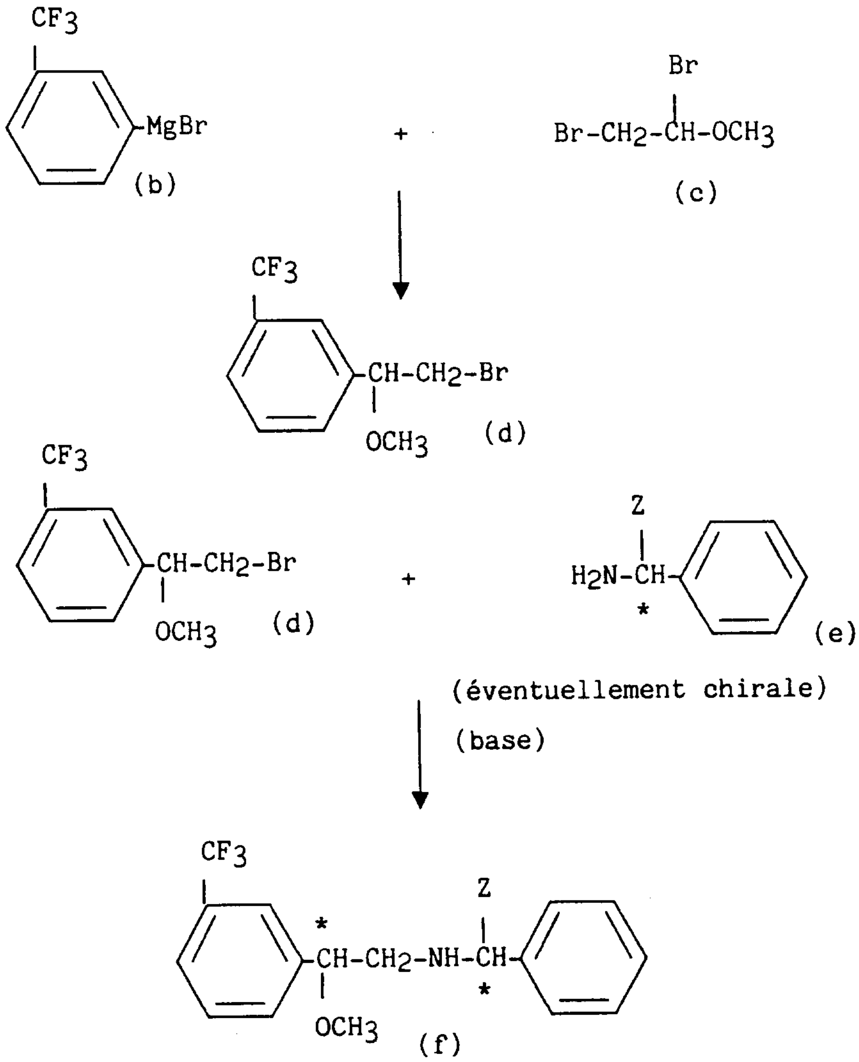


et l'on recristallise cette dernière sous forme de chlorhydrate. Dans le cas où Z représente un radical méthyle, selon que l'α-méthylbenzylamine de départ est R ou S on obtient, par recristallisation, le chlorhydrate du composé (f) sous forme de l'un ou l'autre des diastéréoisomères.

L'amine benzylée de formule f (éventuellement sous forme de diastéréoisomère pur) est ensuite alcoylée au moyen de bromoacétate d'éthyle en présence d'une base telle que le carbonate de potassium pour obtenir le composé de formule g :

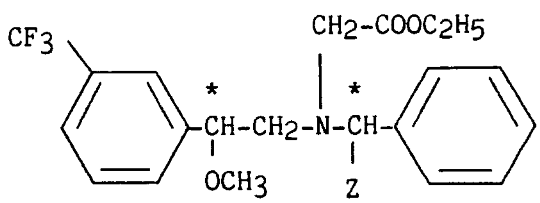

(g)

lequel est alors réduit par $LiAlH_4$ en alcool de formule h :

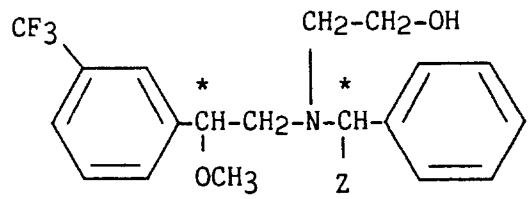

(h)

Ce dernier est ensuite transformé en halogénure III, au moyen d'un composé halogéné (tel que par exemple $SOCl_2$, $PCl_5$ ou $POCl_3$ pour obtenir plus précisément un composé de formule III dans laquelle Hal représente un atome de chlore soit :

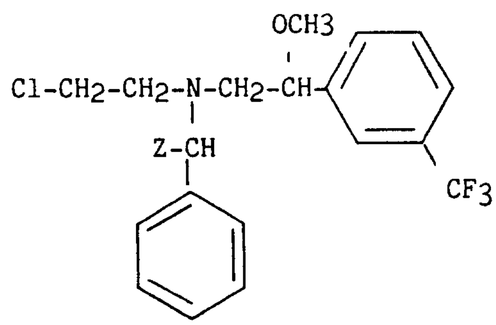

)

La présente invention a aussi pour objet le procédé de préparation des composés de formule générale I caractérisé en ce que :

- l'on fait réagir l'amine primaire (éventuellement chirale) de formule VII :

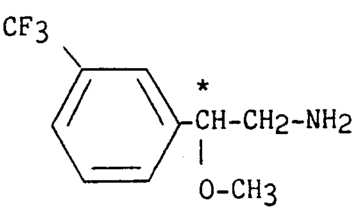

(VII)

avec de l'oxyde d'éthylène pour obtenir le composé de formule VIII :

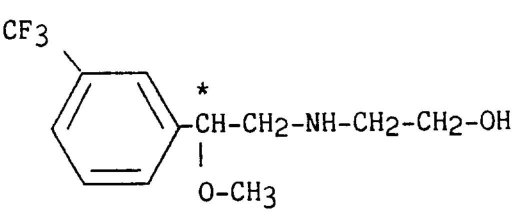

(VIII)

que l'on transforme en chlorure de formule IX :

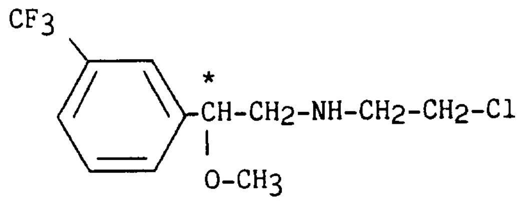

(IX)

et l'on couple ce dernier avec un sel de formule générale II' :

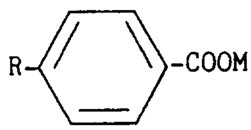

(II')

dans laquelle R et M sont tels que précédemment définis pour obtenir un composé de formule générale I :

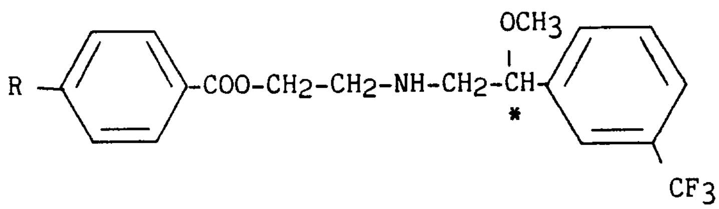

(I)

Les composés de formule générale I peuvent être transformés en sels d'addition avec les acides, sels qui font, à ce titre, partie de la présente invention.

Comme acides utilisables pour la formation de ces sels, on peut citer, par exemple, dans la série minérale, les acides chlorhydrique, bromhydrique, nitrique, sulfurique, phosphorique et dans la série organique, les acides acétique, propionique, maléique, fumarique, tartrique, oxalique, benzoïque, méthane sulfonique et iséthionique.

Les composés de formule générale I et leurs sels d'addition physiologiquement tolérables possèdent des propriétés pharmacologiques et thérapeutiques intéressantes, notamment des propriétés régulatrices du métabolisme des glucides et des lipides. De plus, ils provoquent une diminution modérée de la pression artérielle.

Plus précisémment, les composés de la présente invention améliorent l'efficacité de l'insuline à un niveau périphérique et/ou hépatique, d'où une amélioration de la tolérance au glucose et de d'hyperglycémie modérée, lorsqu'elle existe, sans risque d'hypoglycémie, ainsi qu'une réduction de l'hyperinsulinémie. De plus, les composés réversent l'insulinorésistance induite par l'amyline.

Ils diminuent également l'hypertriglycéridémie et l'opposent à l'oxydation des LDL (Lipoprotéines de faible densité) d'où une implication dans la prévention des macroangiopathies.

Ils provoquent une réduction pondérale modeste, associée à une réduction de prise alimentaire, non reliée à un mécanisme sérotoninergique et par ces deux dernières propriétés, ils se différencient des produits existant dans ce domaine (Benfluorex, Fenfluramine).

Ces propriétés permettent de les utiliser en thérapeutique notamment pour le traitement des diabétiques non insulino dépendants non traités par un régime, des diabétiques non insulino dépendants traités par les médicaments hypoglycémiants, des diabétiques insulino dépendants ou non traités à l'insuline, ou des sujets non hyperglycémiques ayant une hyperinsulinémie (i.e. une obésité androïde) hypertendus ou non et présentant tous une résistance à l'insuline, induite ou non par l'amyline.

Ainsi les produits de l'invention sont utilisés dans le traitement du diabète, de l'obésité, du syndrome X (par le biais de l'amélioration de l'effet de l'insuline à la périphérie et/ou au niveau du foie, de la diminution des triglycérides et de l'oxydation des LDL, associé à une réduction pondécale modérée), et dans le traitement de l'hypertension chez les sujets insulinorésistants ou ayant des anomalies métaboliques telles que, par exemple, hyperinsulinémie, dyslipémie, hyperglycémie, associées ou non, secondaire ou non aux effets de l'amyline.

La présente invention a également pour objet les compositions pharmaceutiques contenant, comme principe actif, un dérivé de formule générale I ou un de ses sels physiologiquement tolérable, mélangé ou associé à un ou plusieurs excipients pharmaceutiques appropriés.

Les compositions pharmaceutiques ainsi obtenues se présentent généralement sous forme dosée contenant de 25 à 100 mg de principe actif. Elles peuvent revêtir la forme de comprimés, dragées, gélules, suppositoires, solutions injectables ou buvables et être administrées par la voie orale, rectale ou parentérale.

La posologie peut varier notablement selon l'âge et le poids du patient, la voie d'administration, la nature de la maladie et les traitements associés et s'échelonne de 25 à 100 mg de principe actif par prise, 1 à 4 fois par jour.

Les exemples suivants illustrent l'invention.

**Exemple 1**

Isomère lévogyre du chlorhydrate de para-[2-(α-fluorén-9-yl acétyl amino) éthyl] benzoate de 2-[(β-méthoxy β-métatrifluorométhylphényl) éthyl amino] éthyle.

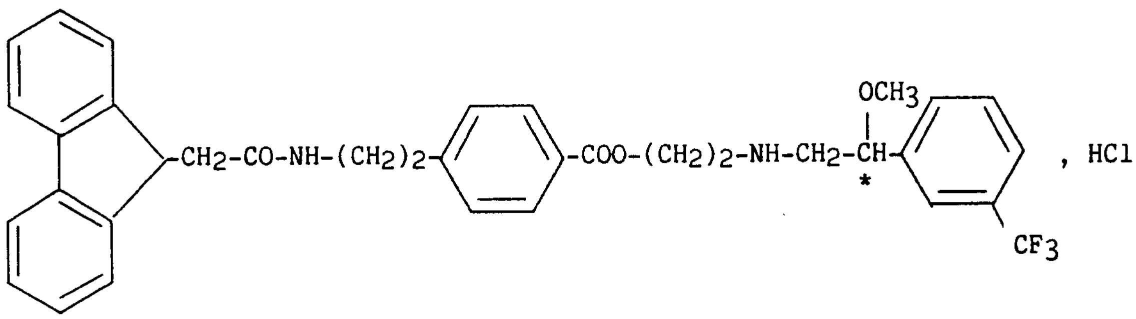

8,3 g de I chlorhydrate de N-(β-chloroéthyl) N(α-phényléthyl) N-[(β-méthoxy β-métatrifluorométhylphényl) éthyl] amine sont agités dans 100 ml d'eau, 100 ml d'éther et 2,5 ml de solution concentrée d'hydroxyde de sodium. Par décantation, la phase ethérée est recueillie, séchée sur $MgSO_4$ ; le solvant est distillé et le résidu dissout dans 50 ml de diméthylformamide anhydre.

Cette solution est alors versée lentement dans un ballon contenant 7,3 g d'acide para[2-(fluorén-9-yl acétylamino) éthyl] benzoïque, 2,7 g de $K_2CO_3$ et 100 ml de diméthylformamide, l'ensemble ayant été préalablement porté à 60 °C pendant 45 minutes.

Après addition du dérivé chloré le mélange réactionnel est porté à 90 °C, sous azote, pendant 3 heures. Le solvant est distillé sous vide et le résidu repris par l'éther. Le précipité de chlorure de potassium est filtré et l'éther distillé. Le résidu obtenu (environ 13 g) est filtré sur 300 g de silice en éluant avec le mélange dichlorométhane/acétate d'éthyle (95/5).

On obtient ainsi 8 g du composé IV de formule :

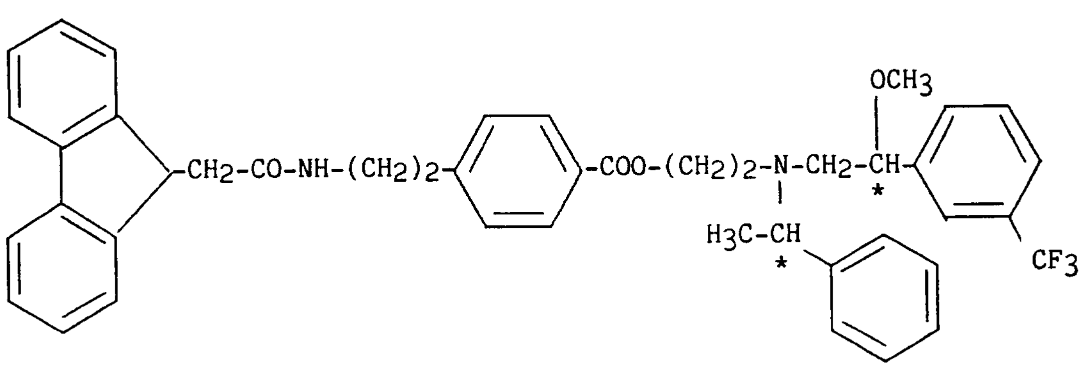

7 g de ce composé mis en solution dans 160 ml d'isopropanol anhydre sont hydrogénés sous une pression d'hydrogène d'environ 90 LBS, jusqu'à absorption totale de la quantité d'hydrogène nécessaire, et ce à une

température de 50 °C.

La solution est ensuite filtrée sur talc, le solvant est distillé et le résidu filtré sur 100 mg de silice en éluant avec un mélange $CH_2Cl_2/CH_3COOC_2H_5$ (50/50).

Le solvant de la fraction contenant le produit est distillé. L'huile résiduelle est reprise par l'éther et additionnée d'un léger excès d'une solution d'éther chlorhydrique. Le précipité obtenu est filtré, lavé à l'éther. On obtient ainsi 4,5 g de l'isomère lévogyre du chlorhydrate de para[2-(α-fluorén-9-yl acétyl amino) éthyl] benzoate de 2-[(β-méthoxy-β-metatrifluorométhylphényl) éthylamino] éthyle.

Pouvoir rotatoire : (C = 1 % dans l'éthanol).

[α]589 : -29,7
[α]578 : -30,6
[α]546 : -34,7
[α]436 : -58,3
[α]365 : -90,9

L'acide para[2-(fluorén-9-yl acétylamino) éthyl] benzoïque de départ a été préparé selon le brevet US 4.136.197.

Le I chlorhydrate de N-(β-chloroéthyl) N-(α-phényléthyl) N-[(β-méthoxy-β-métatrifluorométhylphényl) éthyl] amine de départ a été préparée comme suit :

**a)** 225 g de 3-trifluorométhyl bromobenzène sont additionnés, goutte à goutte dans un mélange de 27 g de magnésium et 500 ml d'éther anhydre auquel on a préalablement ajouté un cristal d'iode. La réaction est effectuée de manière à assurer un léger reflux de l'éther. Une fois le magnésium dissout, on ajoute lentement 232 g de 1-méthoxy 1,2-dibromoéthane (préparé selon la technique décrite dans Organic Synthèses vol IV p 748 en remplaçant l'éthanol par le méthanol).

Le reflux est maintenu pendant 1 heure après la fin de l'addition. Le mélange est alors refroidi puis hydrolysé par 250 ml d'eau. Après décantation, la phase éthérée est lavée à l'eau, puis séchée.

On distille le solvant, puis le produit sous pression réduite. On obtient 203 g de 3-trifluorométhyl 1-(2-bromo 1-méthoxy éthyl) benzène. ($Eb/_{13\ Pa}$ = 68 °C).

**b)** On porte à reflux 202 g de ce dérivé bromé et 173 g d'α-méthyl benzylamine (S) dans 714 ml de xylène pendant 6 heures.

On distille ensuite le xylène et reprend le résidu à l'éther. Le filtrat obtenu, après élimination du bromure d'α-méthylbenzylammnonium, est distillé. On recueille ainsi 144 g de produit qui bout à 125-128 °C sous 13 Pa.

Ce composé dissout dans l'éther est additionné de 94 ml d'éther chlorhydrique 5N. Le précipité obtenu est filtré, lavé à l'éther, recristallisé deux fois dans, chaque fois, 550 ml d'isopropanol.

On obtient ainsi 80 g du diastéréoisomère pur.

**c)** 21 g de ce dernier sont mis à réagir avec 12 g de bromoacétate d'éthyle et 9,8 g de carbonate de potassium dans 65 ml d'éthanol anhydre. On porte à reflux pendant 8 heures, rajoute alors 2 ml de bromoacétate d'éthyle et 2,5 g de carbonate de potassium, et chauffe de nouveau pendant 4 heures. On filtre le dérivé minéral, distille l'éthanol et le bromoacétate en excès, et utilise l'huile résiduelle telle quelle.

**d)** 27 g de l'ester ainsi obtenu sont mis à réagir avec 3,78 g de $LiAlH_4$ dans 150 ml de tétrahydrofurane anhydre, à reflux pendant 5 heures.

On hydrolyse ensuite le mélange par 3,8 ml d 'eau, puis 3,8 ml de NaOH 4N et enfin 11,4 ml d'eau.

On filtre et concentre la phase organique que l'on utilise telle quelle.

**e)** 22,9 g de l'alcool ainsi obtenu sont dissous dans 65 ml de chloroforme. On ajoute goutte à goutte, à la solution, 10,5 ml d'éther chlorhydrique 6N. On évapore le solvant, reprend le résidu dans 65 ml de chloroforme et y ajoute, goutte à goutte, 8,2 g de chlorure de thionyle.

On porte à reflux pendant 5 heures, puis distille le solvant. Le résidu est repris par 150 ml d'acétate d'éthyle et dissous à chaud. On laisse refroidir jusqu'à formation d'un léger précipité correspondant à :

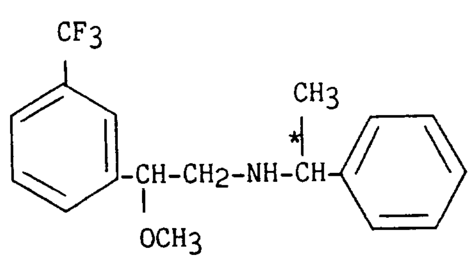 , HCl

que l'on élimine de cette manière.

On distille ensuite le solvant de la solution restante et le produit résiduel (qui est le I chlorhydrate de N-(β-chloroéthyl) N-(α-phényléthyl) N-[(β-méthoxy-β-métatrifluorométhylphényl) éthyl] amine) est utilisé tel quel.

**Exemples 2 à 7**

En opérant comme décrit dans l'exemple 1 ont été préparés les composés suivants :

**2.** Isomère dextrogyre du chlorhydrate de para-[2-(α-fluorén-9-yl acétyl amino) éthyl] benzoate de 2-[(β-méthoxy-β-métatrifluorométhylphényl) éthylamino] éthyle, de pouvoir rotatoire (c = 1% dans l'éthanol).
[α]589 : + 30,0
[α]578 : + 31,3
[α]546 : + 35,8
[α]436 : + 61,4
[α]365 : + 95,0

**3.** dl chlorhydrate de para-[2-(α-florén-9-yl acétylamino) éthyl] benzoate de 2-[(β-méthoxy-β-métatrifluorométhylphényl) éthylamino] éthyle.

**4.** Isomère lévogyre du chlorhydrate de para-[2-(benzhydrylacétyl amino) éthyl] benzoate de 2-[(β-méthoxy-β-métatrifluorométhylphényl) éthylamino] éthyle.
Pouvoir rotatoire (c = 1% dans l'éthanol à 21 °C).
[α]589 : - 32,3
[α]578 : - 33,5
[α]546 : - 38,1
[α]436 : - 64,5
[α]365 : - 100,7.

**5.** dl chlorhydrate du benzoate de 2-[(β-méthoxy-β-métatrifluorométhylphényl) éthylamino] éthyle, PF (Kofler) : 122-123 °C.

**6.** Isomère lévogyre du chlorhydrate du benzoate de 2[(β-méthoxy-β-métatrifluorométhylphényl) éthylamino] éthyle.
Pouvoir rotatoire (c = 1% dans l'éthanol à 23 °C).
[α]589 : - 52,9
[α]578 : - 55,1
[α]546 : - 62,7
[α]436 : - 106,6
[α]365 : - 167,3.

**7.** Isomère dextrogyre du chlorhydrate du benzoate de 2-[(β-méthoxy-β-métatrifluoro-méthylphényl) éthylamino] éthyle.
Pouvoir rotatoire (c = 1% dans l'éthanol à 23 °C).
[α]589 : + 51,5
[α]578 : + 53,9
[α]546 : + 61,1
[α]436 : + 103,6
[α]365 : + 152,2.

**Exemples 8 et 9**

Les matières premières nouvelles de formule générale IIa ont été préparées selon la méthode illustrée dans les exemples suivants :

**8.** Acide para{2-[N-(bis 4,4'-n.pentyloxybenzhydrylacétyl) amino] éthyl} benzoïque

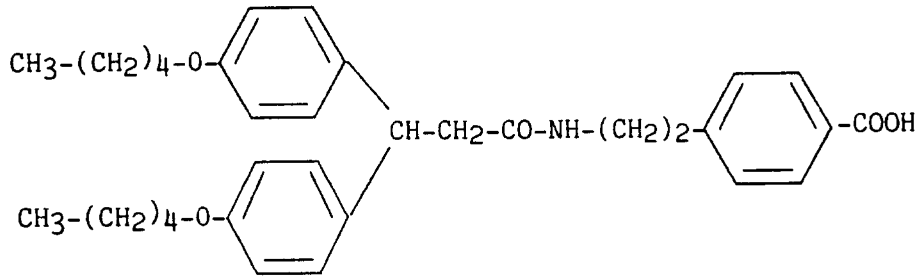

**a)** para n.pentyloxybromobenzène : A 173 g de p.bromophénol en solution dans 1800 ml de méthanol sont ajoutés 276 g de carbonate de potassium sec. Après agitation, le mélange réactionnel est traité avec 164 ml de n.bromopentane et maintenu à reflux pendant 4 heures sous agitation.

Après refroidissement, 3 litres d'eau sont ajoutés et le méthanol est éliminé sous vide de la trompe à eau.

Après extraction par 2 litres puis 2 fois 1 litre d'éther, les solutions organiques sont réunies et lavées par 3 fois 1 litre d'eau. Après séchage sur $MgSO_4$ anhydre, le solvant est évaporé sous vide et le résidu distillé.

On obtient 222,8 g de para-n.pentyloxy bromobenzène ($Eb/_{13\ Pa}$ = 87-91 °C). Rendement : 91,6 %.

**b)** para-n.pentyloxybenzaldéhyde : en opérant comme précédemment à partir de 33 g de para hydroxy benzaldéhyde, 74,5 g de carbonate de potassium et 45 g de n.bromopentane, on a isolé 21 g de para n.pentyloxybenzaldéhyde ($Eb/_{1,3\ Pa}$ : 112-114 °C). Rendement : 37 %.

**c)** n.pentyloxybenzylidène malonate d'éthyle :

A 38,5 g de para-n.pentyloxy benzaldéhyde et 31 ml de malonate d'éthyle en solution dans 400 ml de diméthylformamide anhydre sont ajoutés 32,6 g de chlorhydrate de diméthylamine et 1,7 g de fluorure de potassium. Le mélange réactionnel est chauffé sous agitation pendant 18 heures. Après refroidissement, le solvant est évaporé sous vide ($13.10^2$ à $20.10^2$ Pa). Le résidu est repris par 2 fois 250 ml de chlorure de méthylène. La couche organique est lavée par 200 ml d'une solution normale d'acide chlorhydrique puis 2 fois 200 ml d'eau.

Après séchage sur sulfate de magnésium anhydre, le solvant est évaporé sous vide de la trompe à eau. Par distillation du résidu, on obtient 49,8 g de para-n.pentyloxybenzylidène malonate d'éthyle ($Eb/_{13\ Pa}$ = 170-175 °C). Rendement : 74,5 %.

**d)** $\alpha$-carbéthoxy $\beta,\beta$-[bis(para-n.pentyloxyphényl)] propanoate d'éthyle :

A la solution de bromure de para-n.pentyloxyphényl magnésium préparée à partir de 5,8 g de para-n.pentyloxy bromobenzène, 0,64 g de magnésium en tournures dans 30 ml de tétrahydrofurane (THF) (coulé en 1 heure à reflux puis maintenu à reflux pendant encore 1 heure), sont ajoutés : 8 g de para-n.pentyloxy benzylidène malonate d'éthyle en solution dans 25 ml de THF en 30 minutes. Après 2 heures 30 de reflux sous agitation, on coule 25 ml d'une solution normale d'HCl. Le mélange réactionnel est extrait par trois fois 50 ml d'eau, puis séchée sur $MgSO_4$. Après évaporation, le résidu est purifié par chromatographie. On obtient 8,7 g de produit attendu.

**e)** Acide $\beta,\beta$-[bis(para-n.pentyloxyphényl)] propionique :

A une solution de 6,2 g de potasse dans 100 ml d'eau sont ajoutés 8,5 g de produit obtenu en d) en solution dans 22,5 ml d'éthanol. La solution obtenue est maintenue à reflux pendant 1 heure 30. Après élimination du solvant sous vide de la trompe à eau, le résidu est repris par 200 ml d'eau puis acidifié par 11 ml d'une solution à 37 % d'HCl concentré. L'huile formée est extraite par 3 fois 100 ml d'éther. Après lavage par 2 fois 50 ml d'eau, l'éther est séché sur sulfate de magnésium puis évaporé sous vide. On obtient 5,6 g de produit, PF : 134-136 °C, Rendement : 75 %.

Le résidu est chauffé pendant 15 minutes à 180-190 °C. Après lavage à l'éther de pétrole, on obtient 3,3 g d'acide $\beta,\beta$-[bis(para-n.pentyloxyphényl)] propionique , PF : 72 °C, Rendement : 67 %.

**f)** para- {2-[N-(bis 4,4'-n.pentyloxy benzhydryl acétyl) amino] éthyl} benzoate d'éthyle :

A 3,06 g de l'acide préparé en e) en solution dans 30 ml de THF, sont ajoutés 1,07 ml de triéthylamine dans 10 ml de THF. Après 1 heure d'agitation, la solution obtenue est ajoutée à 0,76 ml de chloroformiate d'éthyle dans 30 ml de THF refroidi à 0 °C. Puis 1,56 g de para[$\beta$(amino) éthyl] benzoate d'éthyle en solution dans 10 ml de THF sont coulés en 15 minutes. La température s'élève à 10 °C. Après 45 minutes d'agitation à température ambiante, et 12 heures de reflux, le précipité est

essoré et le filtrat tiré à sec sous vide. Après chromatographie, on obtient 3,06 & du benzoate attendu.

**g)** Acide para-{2-[N-(bis 4,4'-n.pentyloxy benzhydryl acétyl) amino] éthyl} benzoïque :

3 g de l'ester obtenu en f), 15 ml d'éthanol et 5,7 ml de solution normale de soude maintenus à reflux pendant 2 heures, conduisent à l'acide benzoïque correspondant.

**9.** Acide para-{2-[N-(3-méthyl 3,3-diphényl propionyl) amino] éthyl} benzoïque :

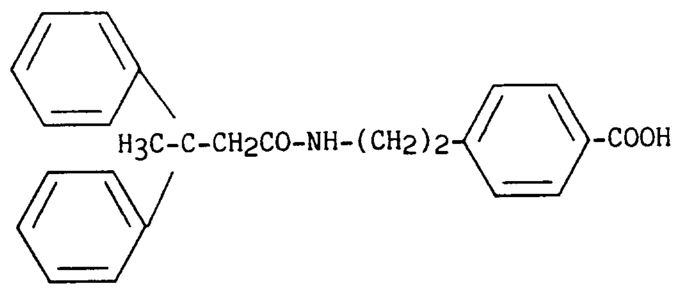

**a)** ($\alpha$-méthylbenzylidène) cyanacétate d'éthyle :

60 g d'acétophénone, 56,6 g de cyanacétate d'éthyle, 7,7 g d'acétate d'ammonium, 24 g d'acide acétique et 100 ml de benzène sont mis à reflux sous agitation et l'eau formée au cours de la réaction est éliminée au fur et à mesure à l'aide d'un Dean Stark.

Après 13 heures 40 de chauffage, 100 ml d'éther sont ajoutés et le mélange reactionnel est lavé par 3 fois 100 ml d'eau. La couche organique est séchée sur sulfate de magnésium, concentrée sous vide de la trompe à

eau et le résidu est distillé. On obtient ainsi 58,4 g d'($\alpha$-méthylbenzylidène) cyanacétate d'éthyle ($Eb/_{13\ Pa}$ = 125-128 °C).

**b)** $\alpha$-carbéthoxy $\beta$,$\beta$-diphényl butyronitrile :

A 6,3 g de magnésium en tournures et 240 ml d'éther anhydre, sont ajoutés, sous agitation en 1 heure 20, 41 g de bromobenzène dissous dans 100 ml d'éther anhydre. Après 2 heures de reflux, on ajoute en 30 minutes 51 g d'($\alpha$-méthylbenzylidène) cyanacétate d'éthyle dans 150 ml d'éther. Après 5 heures de chauffage, le mélange réactionnel est hydrolysé par 150 ml d'HCl, 2N. La couche organique est décantée. La couche aqueuse est extraite par 2 fois 100 ml d'éther. Les phases éthérées sont réunies, lavées par 100 ml d'eau, séchées sur $MgSO_4$ et après filtration concentrées sous vide de la trompe à eau.

Par distillation du résidu, on obtient 30,7 g du produit attendu ($Eb/_{13\ Pa}$ = 150-155 °C), Rendement : 46 %.

**c)** $\alpha$-carboxy $\beta$,$\beta$-pdiphényl butyronitrile :

15 g d'$\alpha$-carbéthoxy $\beta$,$\beta$-diphényl butyronitrile, 65 ml d'éthanol, 18,4 g de potasse en pastilles et 30 ml d'eau sont agités à température ambiante pendant 4 heures. Après concentration sous vide, le résidu est repris par 150 ml d'eau. Après extraction par 3 fois 50 ml d'éther, la couche aqueuse est acidifiée par 22 ml d'HCl concentré et extraite par 3 fois 100 ml d'éther. Les couches organiques sont réunies, séchées sur $MgSO_4$ et évaporées. On obtient 14 g du produit attendu.

**d)** $\beta$,$\beta$-diphénylbutyronitrile :

14 g d'$\alpha$-carboxy $\beta$,$\beta$-diphénylbutyronitrile et 85 ml de triéthylène glycol sont chauffés pendant 30 minutes à 80 °C, puis, après refroidissement, dilués avec 300 ml d'eau. Par extraction par 3 fois 150 ml d'éther, séchage de la couche organique et concentration sous vide on obtient, après purification par chromatographie, 7,3 g de $\beta$,$\beta$-diphénylbutyronitrile. Rendement : 64 %.

**e)** Acide $\beta$,$\beta$-diphénylbutyrique :

7,2 g du produit obtenu en d), 9,6 ml d'acide sulfurique concentré, 12 ml d'eau et 12 ml d'acide acétique sont maintenus à reflux pendant 22 heures. Après refroidissement et traitement par 90 ml d'eau, le précipité formé est essoré et lavé à l'eau. Après séchage et recristallisation dans 20 ml de cyclohexane, on obtient 6,5 g de l'acide attendu, PF : 100 °C, Rendement : 77 %.

**f)** chlorure de $\beta$,$\beta$-diphénylbutyryle :

6,5 g de l'acide précédent sont ajoutés en 15 minutes à 10 ml de chlorure de thionyle. Après chauffage 2 heures à reflux, la solution obtenue est concentrée sous vide et le résidu repris par 2 fois 50 ml de benzène, en évaporant à chaque fois. On obtient alors 6,6 g de chlorure de $\beta$,$\beta$-diphénylbutyryle.

**g)** Acide para-{2[N-(3-méthyl 3,3-diphényl propionyl) amino] éthyl} benzoïque :

A une suspension de 5,2 g de chlorhydrate de l'acide para-[$\beta$(amino) éthyl] benzoÏque dans 200 ml de diméthylformamide anhydre sont ajoutés, sous agitation, 31,5 g de triéthylamine, puis 6,5 g du chlorure obtenu en f) en solution dans 75 ml de THF anhydre sont coulés en 15 minutes. La température s'élève de 26 à 34 °C. Après 4 heures d'agitation à température ambiante, puis 5 heures à 40 °C, le chlorhydrate de triéthylamine formé est essoré et le filtrat concentré sous vide. Le résidu est repris par 60 ml d'eau. Le précipité formé est essoré, séché à l'air, puis recristallisé dans 50 ml d'isopropanol anhydre. On obtient 5 g d'acide para-{2-[N-(3-méthyl 3,3-diphényl propionyl) amino] éthyl} benzoïque , PF : 211 °C.

**Exemple 10** :

**ETUDE PHARMACOLOGIOUE**

A. ETUDE DE L'EFFET D'UN TRAITEMENT AIGU ADMINISTRÉ EN PERFUSION PORTALE CHEZ LE RAT VIGILE

**1. But de l'expérimentation**

La technique de perfusion portale chez le rat vigile permet d'étudier l'effet de substances pharmacologiques sur les modifications de la tolérance au glucose adminstré par voie intraveineuse.

L'injection d'agents directement dans la circulation portale permet d'éviter les effets du transit gastrique, d'absorption et de libération d'hormone par la paroi intestinale et ainsi d'explorer :

- soit un effet direct sur le tissu hépatique,
- soit l'existence d'un relai foie-système nerveux central-tissu périphérique (foie, muscle ou cible endocrine).

**2. Protocole**

**2.1 Animaux utilisés**

Dans cette expérimentation, on a utilisé des rats mâles adultes SPRAGUE DAWLEY par lots de 5 à 12 rats.

Les rats utilisés, agés de 52 semaines, présentent

- une diminution de la tolérance au glucose,
- une augmentation de l'insulinémie basale, et
- une augmentation des lipides plasmatiques.

La stabulation (de 9 à 52 semaines) de ces rats a été réalisée dans une pièce à température régulée de 21 à 22 °C et soumise à un cycle fixe de lumière (de 7 h 30 à 19 h 30) et d'obscurité (de 19 h 30 à 7 h 30). Leur alimentation a consisté en un régime d'entretien (UAR A 03) ; eau et nourriture ont été fournies "ad libitum", à l'exception du jeûne nocturne précédant les tests où la nourriture était retirée.

**2.2 Méthodes**

Préparation chirurgicale :

A $J_7$ avant le début de l'expérimentation, les rats sont anesthésiés à la kétamine (IMALGENE 1000 - Rhône MERIEUX), et un cathéter en silastic (602-135, médical grade, DOW CORNING MIDLAND) est implanté dans la veine porte hépatique [cf. Strubbe J.H., Wolsink J.G., Schutte A.M., Balkan B., and Prins A.J.A. : Hepatic-portal and cardiac infusion of CKK-8 and glucagon induce different effects on feeding. Physiology and Behavior 46,643-646 (1989)] pour la perfusion du soluté physiologique (jour contrôle) puis du produit à tester. Ils sont alors placés individuellement dans des cages en plexiglas.

- Après récupération, soit environ 10 jours plus tard, un second cathéter en silastic (602-155, médical grad, DOW CORNING MIDLAND) est introduit jusque dans l'oreillette droite par la veine jugulaire droite [cf. Steffens A.B : A Method for frequent sampling of blood and continuous infusion of fluids in the rat without disturbing the animal. Physiology and behavior 4, 833-836 (1969)]. Lors des test, les différents prélèvements sanguins et l'injection intra-cardiaque du bolus glucosé seront effectués à l'aide de ce cathéter.

Pendant les deux interventions chirurgicales, les cathéters sont glissés sous la peau et extériorisés au-dessus de la tête au moyen d'une aiguille limée recourbée et bouchée par un capuchon de polyéthylène (PE) fermé à la bougie.

- 0,4 ml d'une solution d'héparine à 500 U/ml (ROUSSEL UCLAF) suivie de 0,1 ml de PVP (Solution de Polyvinylpyrrolidone à 30 % - PM 25000 - MERCK) sont introduits dans les cathéters avant la fermeture par le capuchon de polyéthylène.
  La viscosité du PVP permet d'éviter un reflux du sang dans le cathéter.
- La veille de chaque expérience, les rats sont soumis à un jeûne nocturne de 18 heures. Le jour de l'expérience, ces rats sont pesés et le bon fonctionnement des cathéters vérifié.
- Afin d'éliminer le stress du branchement des cathéters sur les aiguilles extériorisées sur le dessus de la tête, 20 minutes de repos sont nécessaires avant de commencer l'expérience.
- Les perfusions intraportales, réalisées à l'aide d'un cathéter en polyéthylène N° 3 durent 60 minutes ; elles ont un débit constant de 2 ml/heure soit envirion 0,033 ml/minute, assuré par un perfuseur BRAUN. Une épreuve d'IVGTT (test de tolérance au glucose par administration intraveineuse) a lieu 30 minutes après le début de la perfusion portale.
- L'expérience contrôle (test de référence) consiste à perfuser une solution saline (chlorure de sodium isotonique 0,9 % BIOSEDRA) qui permettra pour chaque rat de déterminer la valeur basale de tolérance (K) à une charge glucosée.
- L'insulinorésistance est induite chez le rat normal par une perfusion intraportale d'amyline (IAPP, insulinoma associated pancreatic polypeptide) 26 nmol/kg/h pendant 90 minutes.
- Des prélèvements sanguins de 0,40 ml sont recueillis (cathéter en polyéthylène N° 6) immédiatement avant la perfusion et 30 minutes après le début de la perfusion. Ces échantillons servent à la détermination de la glycémie et de l'insulinémie basales. Immédiatement après le recueil de la basale N° 2, c'est à dire 30 minutes après le début de la perfusion, on administre un bolus glucosé (1 ml/kg d'une solution de glucose à 50 %) dans la veine jugulaire. Une série de prélèvements est ensuite réalisée toute les 3 minutes de t+30 à t+60 minutes après le bolus. Un volume de 0,40 ml est prélevé à chaque fois, et remplacé par 0,40 ml de sérum physiologique hépariné.

Le bon fonctionnement des cathéters est garantie par l'injection de PVP entre chaque expérience.

Recueil des échantillons :

- Pour la détermination de la glycémie, le sang est recueilli sur URAC (solution de déprotéinisation) à raison de 20 µl de sang pour 200 µl d'URAC (dilution au 1/10ème).
- Les échantillons destinés au dosage de l'insulinémie sont recueillis sur héparine (20 µl de sérum physiologique hépariné à 500 UI/ml soit 7,5 UI d'héparine par tube à centrifuger).
- Tous les tubes sont placés dans la glace au moment du recueil des prélèvements. Ces derniers sont ensuite centrifugés 10 minutes à 3000 trs/min (centrifugeuse réfrigérante), pour séparer les éléments figurés dans les plus brefs délais.
- Les sérums (dans l'URAC pour la glycémie) et les plasmas (insulinémie) sont ensuite répartis dans des tubes eppendorf mis au congélateur (-20 °C) jusqu'au jour du dosage.

Procédures analytiques :

Détermination de la glycémie :

Le glucose sanguin est mesuré par la technique de la glucose oxydase à l'aide du kit Boehringer.

- Un contrôle de qualité est assuré par la détermination des valeurs de standards et de contrôles.

Mesure de l'assimilation glucidique :

(cf. Conard v. : Mesure de l'assimilation du glucose, bases théoriques et applications cliniques. Les éditions "acta medica belgica" (1959)).

La vitesse de décroissance de la glycémie après surcharge glucosée est exprimée par le coefficient angulaire K de la droite log c = log A - Kt obtenue sur papier semi-logarithmique en portant le temps (min) en abscisse et le ln [glucose] en ordonnée. Ce coefficient est calculé pour les valeurs de glycémies correspondant aux temps situés entre la 6ème et la 30ème minute après le bolus. Ce calcul est effectué par un ordinateur, à l'aide d'un programme de régression linéaire, après vérification par représentation graphique.

Ce coefficient d'assimilation glucidique est considéré comme une constante, caractéristique d'un sujet donné.

Détermination de l'insulinémie :

- L'insuline plasmatique immunoréactive (IRI) est dosée par une méthode radio-immunologique de kit Phadeseph de PHARMACIA, technique en phase solide d'un double anticorps.
- La détermination des standards et des échantillons est effectuée en double.

Préparation des solutions :

- Pour l'expérience contrôle (test de référence) :
  Perfusion intraportale de chlorure de sodium isotonique 0,9 % (BIOSEDRA)
- Pour la perfusion intraportale du réactif pharmacologique : Une solution mère à 1 mg de produit base par ml d'eau distillée est réalisée et sert à la préparation de la solution finale (dans NaCl 0,9 %).

## Résultats

Ils sont répertoriés dans les tableaux Ia et Ib ci-après.

**TABLEAU Ia**

| Rats SDCD mâles 52 semaines | Insulinémie basale µU/ml Variation % | Insulinémie au pic µU/ml Variation % | Insulinémie 30 mn après glucose µU/ml Variation % | Tolérance au glucose $K10^{-2}$ % |
|---|---|---|---|---|
| Contrôle | 24,06 ± 3,03 | 57,4 ± 13,7 | 29,0 ± 3,7 | 2,72 ± 0,23 |
| **Benfluorex** 7,5 µg/kg/min×60 min | - 42% p<0,05 | - 5% NS | - 36% NS | + 50% p=0,025 |
| **Diméthylbiguanide** 15 µg/kg/min×60 min | Inactif | | | |
| **Produit de l'exemple 1** | | | | |
| 0,25 µg/kg/min×60 min | - 42% p<0,05 | 0 | - 21% NS | + 3% NS |
| 0,50 µg/kg/min×60 min | - 6% p<0,001 | - 21% NS | - 69% p<0,001 | + 40% p=0,025 |
| 0,75 µg/kg/min×60 min | - 25% p=0,05 | + 10% NS | - 36% NS | + 60% p=0,005 |

Un cathéter intraportal et un cathéter intracardiaque (oreillette droite) sont implantés chez des rats SDCD agés de 52 semaines. Après récupération post chirurgicale, les différents groupes de rats sont soumis à un jeûne de 18 heures. Les rats sont éveillés pendant l'expérience. Les produits sont administrés par perfusion intraportale pendant 1 heure. Une épreuve d'hyperglycémie provoquée est pratiquée 30 minutes après le début de la perfusion qui se poursuit pendant l'IVGTT.

Tableau Ib

INSULINORÉSISTANCE INDUITE PAR UNE PERFUSION D'AMYLINE INTROPORTALE

(AUX RATS AGÉS DE 12 SEMAINES)

| | Insulinémie basale(µU/ml) | Insulinémie au pic (µU/ml) | Insulinémie 24 min après glucose(µU/ml) | Tolérance au glucose K.10-2 |
|---|---|---|---|---|
| Controle | 5,93±0,48 | 24,80±1,86 | 8,86±0,77 | 3,54±0,24 |
| Perfusion amyline | 6,87±0,77 | 23,54±1,7 | 15,28±1,90 | 1,71±0,27 |
| Perfusion amyline + produit de l'exemple 1 (0,75µg/kg/min pendant 60 mn) | 8,26±1,15 | 23,60±2,17 | 15±1,86 | 3,79±0,38 |

## B. ETUDE DE L'EFFET D'UN TRAITEMENT CHRONIOUE ADMINSTRÉ PER OS AU RAT SDCD MÂLE AGÉ DE 52 SEMAINES :

### 1. But de l'expérimentation

Les essais sont réalisés sur des rats qui présentent des anomalies pondérales associées à un hyperinsulisme et une hyperglycéridémie.

Il est recherché :

- d'une part l'effet d'un traitement prolongé du produit de l'exemple 1 et de substances de référence sur ces anomalies, et
- d'autre part, la consommation du glucose par le tissu adipeux est mesuré à l'état basal et en présence d'insuline ($10^{-9}$ M).

### 2. Protocole

#### 2.1 Animaux utilisés

On utilise des rats identiques à ceux de l'étude du traitement aigu, (cf. ci-dessus le paragraphe A- 2.1), à savoir des rats SDCD mâles agés de 52 semaines.

#### 2.2 Méthodes

9 jours avant le début de l'expérimentation ($J_{-9}$), sont constitués des lots de rats par randomisation stratifiée sur le poids.

5 jours avant le début de l'expérimentation ($J_{-5}$), les rats sont conditionnés par administration d'une solution de gomme.

Le premier jour de l'expérimentation ($J_1$), les produits à tester sont administrés à différentes doses aux rats 2 fois par jour. Plus précisémment, les produits sont administrés en suspension dans la gomme entre 9 et 10 heures et 16-17 heures pendant 14 jours. Une pesée quotidienne des animaux traités est effectuée.

Le 15ème jour, les rats (à jeun depuis 18 heures) sont sacrifiés par décapitation. Le sang est recueilli directement dans une cupule. Un aliquote (50 µl) est transféré dans 500 µl d'acétate d'uranyl pour dosage de la glycémie. Un aliquote de 3 ml est transféré dans un tube contenant une solution d'héparine (30 µl pour 1 ml de sang total) et centrifugé pour séparer le plasma. Un autre aliquote de 300 µl est transféré dans un tube contenant 15 µl d'une solution d'EDTA/NaF pour dosage des lactates.

Le tissu adipeux épididymaire est prélevé pour étude métabolique immédiatement après sacrifice.

Pour chaque animal, deux fragments de tissu épididymaire droit et gauche sont émincés aux ciseaux et répartis dans 6 fioles d'incubation. Trois de ces fioles contenant 500 µl de milieu et les autres 500 µl de milieu additionné d'insuline de porc ($10^{-9}$ M), la production de $CO_2$ est donc mesurée en triplicate pour chacun des rats de chaque groupe.

## 2.3 Résultats

Ils sont répertoriés dans le tableau II ci-après.

Tableau II

| Traitement Rats SDCD mâles de 52 semaines | Variation pondérale % | Insulinémie basale µU/ml variation % | Glycémie g/l variation % | Tolérance au glucose K $10^{-2}$ variation % | Triglycéride g/l variation % | Cholestérol g/l variation % | Sérotonine cérébrale |
|---|---|---|---|---|---|---|---|
| Contrôle | + 0,4 % | 32 ± 2,4 | 1,06 ± 0,05 | 3,01 ± 0,24 | 3,64 ± 0,57 | 1,1 ± 0,14 | |
| **Benfluorex** | | | | | | | |
| 1 mg/kg×2 | - 2 % (NS) | - 10% NS | - 10% NS | + 30% p<0,05 | - 43 % p=0,025 | - 20% NS | Diminuée |
| 2,5 mg/kg×2 | - 6 % p=0,85 | - 16% NS | - 12% NS | + 25% p=0,05 | - 44 % p=0,05 | - 30% NS | Diminuée |
| **Produit de l'exemple 1** | | | | | | | |
| 0,5 mg/kg×2 | - 1,4 % p=0,005 | - 12 % p=0,5 | - 5% NS | + 26 % (NS) p=0,088 | - 18 % (NS) | 0 | non modifiée |
| 1,0 mg/kg×2 | - 1,9 % p=0,005 | - 20 % p=0,2 | + 10% NS | + 41 % p=0,064 | - 38 % | - 8 % (NS) | non modifiée |
| 2,5 mg/kg×2 | - 3,6 % p=0,005 | - 41 % p=0,005 | - 10% NS | + 59 % p=0,025 | - 50 % p=0,01 | - 34 % p=0,025 | non modifiée |

## C- ETUDE DE L'OXYDATION DES LDL IN VITRO

Une étude comparative entre le produit de l'exemple 1 et les substances de référence (Benfluorex, Diméthylbiguanide) sur l'oxydation des LDL in vitro a été réalisée.

Les résultas sont répertoriés dans le tableau III ci-après.

Tableau III

| | **Oxydation des LDL in vitro** | | **Incorporation d'oléate dans les esters de cholestérol** |
|---|---|---|---|
| | par le cuivre | par les monocytes | |
| **Benfluorex** | Inactif à $10^{-4}$ M | Inactif à $10^{-4}$ M | - 30 % à $10^{-5}$ M |
| **Diméthylbiguamide $10^{-4}$ M** | Inactif à $10^{-4}$ M | Inactif à $10^{-4}$ M | Inactif à $10^{-5}$ M |
| **Produit de l'exemple 1** | $IC_{50}$ = $10^{-5}$ M | $IC_{50}$ = $10^{-5}$ M | - 70 % à $10^{-5}$ M |

## D- RECHERCHE D'UN EFFET HYPOTENSEUR CHEZ LE CHIEN ÉVEILLÉ

La pression artérieile a été mésurée par brassard externe (Sphygmotensiomètre) au niveau de la queue du chien, avant et après traitement avec le produit de l'exemple 1 à la dose de 5 mg/kg P.O.

Les résultats sont répertoriés dans le tableau IV ci-après.

Tableau IV

| **Produit testé** | **Animal traité (nombre : n)** | **Diminution de la pression artérielle (PA)** | |
|---|---|---|---|
| | | **PA Systolique** | **PA Diastolique** |
| Produit de l'exemple 1 (5 mg/kg P.O) | chien (n = 4) | -2,4.$10^3$ Pa à -3,6.$10^3$ Pa | -3.$10^3$ Pa à -5.$10^3$ Pa |

## E. CONCLUSION

Les résultats des études ci-dessus décrites montrent l'intérêt pharmacologique et thérapeutique de l'isomère lévogyre du chlorhydrate de para-[2-(α-fluorén-9-yl acétyl amino) éthyl] benzoate de 2-[(β-méthoxy β-métatrifluorométhylphényl) éthyl amino] éthyle (produit particulièrement représentatif des dérivés de la présente invention) et l'originalité et la supériorité de ce dernier vis à vis de substances de référence reconnues comme spécifiquement adaptées aux traitements concernés.

## Revendications

**Revendications pour les Etats contractants suivants : AT, BE, CH, DE, DK, FR, GB, IT, LI, LU, NL, PT, SE**

**1.** Les composés de benzoate d'éthanolamine de formule générale I :

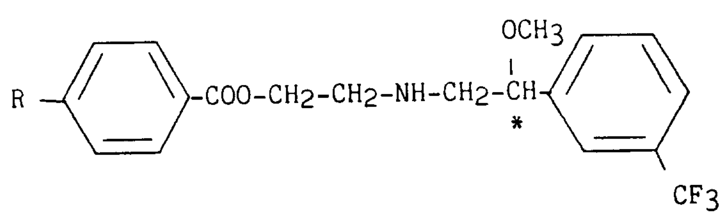

(I)

dans laquelle :

R représente :

- un atome d'hydrogène, ou
- un groupe de formule :

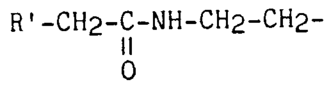

dans laquelle R' représente :

**a)** soit un radical de formule :

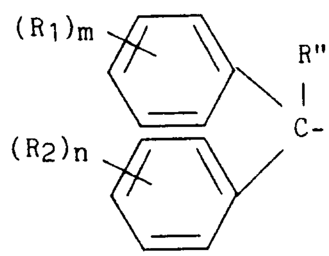

dans laquelle :

R'' représente un atome d'hydrogène ou un radical alkyle contenant de 1 à 5 atomes de carbone en chaine droite ou ramifiée,

$R_1$ et $R_2$, identiques ou différents, représentent chacun un atome d'hydrogène ou un radical alkyle ou alkoxy ayant chacun de 1 à 5 atomes de carbone en chaine droite ou ramifiée, et m et n identiques ou différents représentent chacun 1, 2 ou 3 ;

**b)** soit un radical fluorényle de formule :

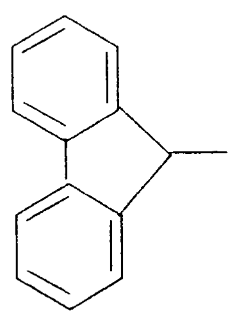

sous forme de composé racémique ou d'énantiomères.

**2.** Les sels physiologiquement tolérables des composés de la revendication 1 avec des acides appropriés.

**3.** Le l chlorhydrate de para-[2-(α-fluorén-9-yl acétyl amino) éthyl] benzoate de 2-[(β-méthoxy β-métatrifluorométhylphényl) éthylamino] éthyle.

**4.** Le d chlorhydrate de para-[2-(α-fluorén-9-yl acétyl amino) éthyl] benzoate de 2-[(β-méthoxy β-métatrifluorométhylphényl) éthylamino] éthyle.

**5.** Le dl chlorhydrate de para-[2-(α-fluorén-9-yl acétyl amino) éthyl] benzoate de 2-[(β-méthoxy β-métatrifluorométhylphényl) éthylamino] éthyle.

**6.** Le l chlorhydrate de para-[2-(benzhydrylacétylamino) éthyl] benzoate de 2-[(β-méthoxy β-métatrifluorométhylphényl) éthylamino] éthyle.

**7.** Le dl chlorhydrate du benzoate de 2-[(β-méthoxy β-métatrifluorométhylphényl) éthylamino] éthyle.

**8.** Le l chlorhydrate du benzoate de 2-[(β-méthoxy β-métatrifluorométhylphényl) éthylamino] éthyle.

**9.** Le d chlorhydrate du benzoate de 2-[(β-méthoxy β-métatrifluorométhylphényl) éthylamino] éthyle.

**10.** Le procédé de préparation des composés de la revendication 1 caractérisé en ce que :

- l'on transforme l'acide de formule générale II :

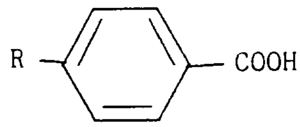

(II)

dans laquelle R a la signification définie dans la revendication 1, en un sel de formule générale II' :

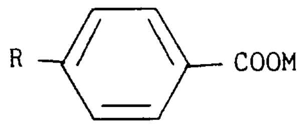

(II')

dans laquelle R est tel que ci-dessus défini et M est un métal alcalin ou alcalino-terreux ;

- l'on fait réagir ce dernier avec un composé halogéné de formule générale III :

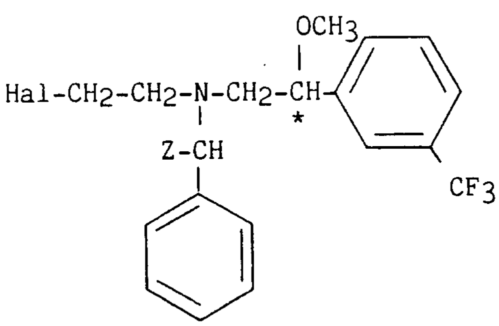

(III)

dans laquelle

- Z représente un atome d'hydrogène ou un radical méthyle, et
- Hal représente un atome d'halogène ;
- et l'on débenzyle catalytiquement le composé ainsi obtenu de formule générale IV :

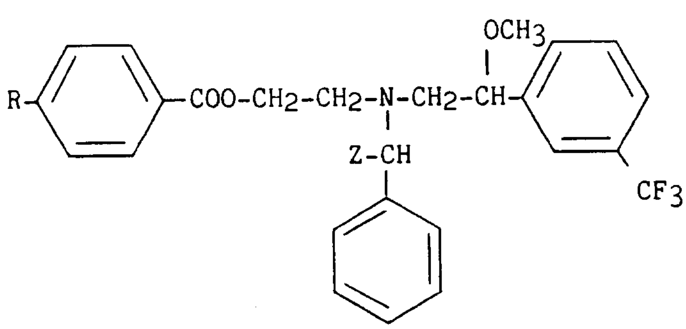

(IV)

dans laquelle R et Z sont tels que précédemment définis.

**11.** Les compositions pharmaceutiques contenant comme principe actif un composé selon les revendications 1 à 9 avec un ou plusieurs excipients pharmaceutiques appropriés.

**12.** Les compositions pharmaceutiques selon la revendication 11 présentées sous une forme convenant notamment dans le traitement du diabète, de l'obésité, du syndrome d'insulinorésistance, "Syndrome X", secondaire ou non à une hyperamylinémie associant une diminution de la tolérance au glucose, hyperinsulinémie, dyslipémie et hypertension, et dans le traitement de l'hypertension chez les sujets insulinorésistants ou ayant une ou plusieurs anomalies métaboliques.

**Revendication pour les Etats contractants suivants : ES, GR**

**1.** Procédé de préparation des composés de benzoate d'éthanolamine de formule générale I :

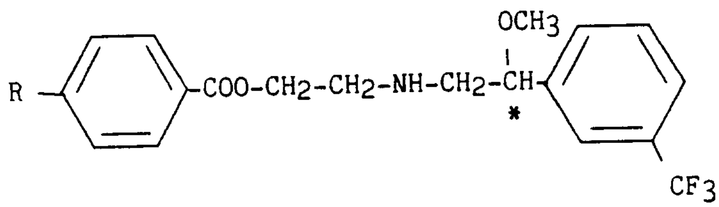

(I)

dans laquelle :

R représente :

- un atome d'hydrogène, ou
- un groupe de formule :

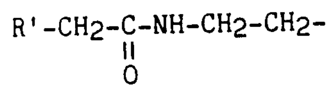

dans laquelle R' représente :

**a)** soit un radical de formule :

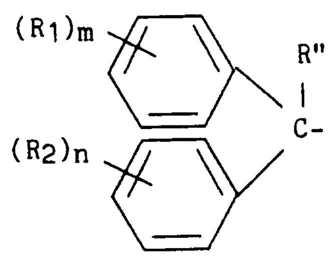

dans laquelle :

R'' représente un atome d'hydrogène ou un radical alkyle contenant de 1 à 5 atomes de carbone en chaine droite ou ramifiée,

$R_1$ et $R_2$, identiques ou différents, représentent chacun un atome d'hydrogène ou un radical alkyle ou alkoxy ayant chacun de 1 à 5 atomes de carbone en chaine droite ou ramifiée, et m et n identiques ou différents représentent chacun 1, 2 ou 3 ;

**b)** soit un radical fluorényle de formule :

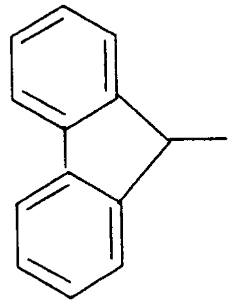

sous forme de composé racémique et d'énantiomères,
et de leurs sels physiologiquement tolérables avec des acides appropriés.
caractérisé en ce que :

- l'on transforme l'acide de formule générale II :

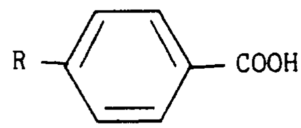

(II)

dans laquelle R a la signification précédemment définie,
en un sel de formule générale II' :

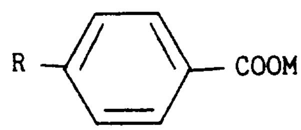

(II')

dans laquelle R est tel que ci-dessus défini et M est un métal alcalin ou alcalino-terreux ;

- l'on fait réagir ce dernier avec un composé halogéné de formule générale III :

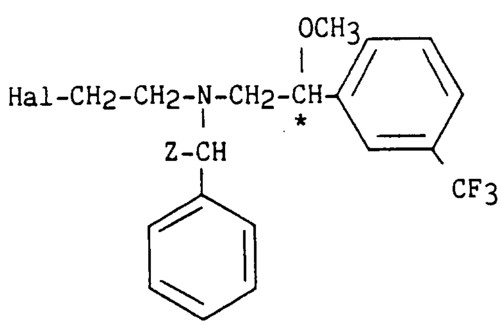

(III)

dans laquelle

- Z représente un atome d'hydrogène ou un radical méthyle, et
- Hal représente un atome d'halogène ;

- et l'on débenzyle catalytiquement le composé ainsi obtenu de formule générale IV :

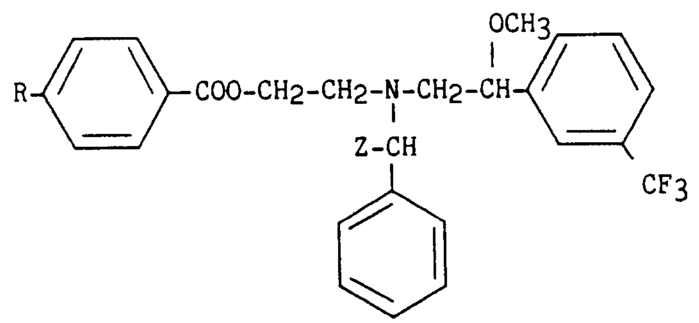

(IV)

dans laquelle R et Z sont tels que précédemment définis,

et si on le désire, les composés de formule I ainsi obtenus sont traités avec des acides physiologiquement tolérables pour donner les sels d'addition acides correspondants.

**Claims**

**Claims for the following Contracting States : AT, BE, CH, DE, DK, FR, GB, IT, LI, LU, NL, PT, SE**

1. Ethanolamine benzoate compounds of the general formula I:

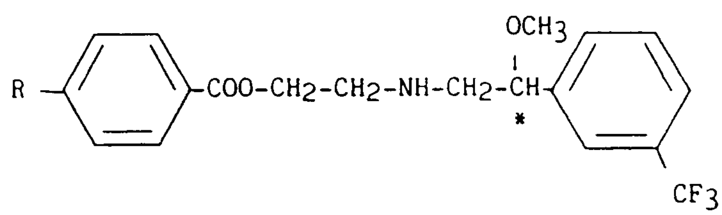

**(I)**

wherein:

R represents:

- a hydrogen atom or
- a group of the formula:

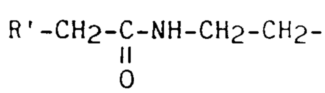

wherein R' represents:

a) either a radical of the formula:

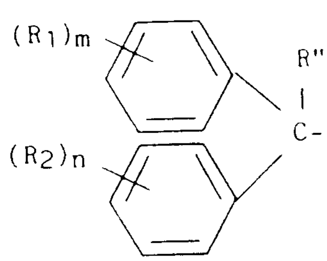

wherein:

R" represents a hydrogen atom or an alkyl radical containing from 1 to 5 carbon atoms in a straight or branched chain,

each of $R_1$ and $R_2$, which may be identical or different, represents a hydrogen atom or an alkyl or alkoxy radical each having from 1 to 5 carbon atoms in a straight or branched chain, and

each of m and n, which may be identical or different, represents 1, 2 or 3;

b) or a fluorenyl radical of the formula:

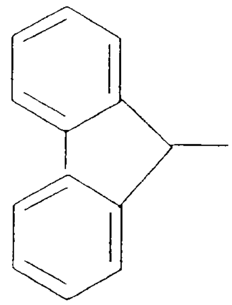

in the form of racemic compounds or enantiomers.

**2.** The physiologically tolerable salts of the compounds of claim 1 with appropriate acids.

**3.** l-2-[(β-methoxy-β-meta-trifluoromethylphenyl)ethylamino]ethyl para-[2-(α-fluoren-9-ylacetylamino)ethyl] benzoate hydrochloride.

**4.** d-2-[(β-methoxy-β-meta-trifluoromethylphenyl)ethylamino]ethyl para-[2-(α-fluoren-9-ylacetylamino)-ethyl] benzoate hydrochloride.

**5.** dl-2-[(β-methoxy-β-meta-trifluoromethylphenyl)ethylamino]ethyl para-[2-(α-fluoren-9-ylacetylamino)-ethyl] benzoate hydrochloride.

**6.** l-2-[(β-methoxy-β-meta-trifluoromethylphenyl)ethylamino]ethyl para-[2-(benzhydrylacetylamino)ethyl] benzoate hydrochloride.

**7.** dl-2-[(β-methoxy-β-meta-trifluoromethylphenyl)ethylamino]ethyl benzoate hydrochloride.

**8.** l-2-[(β-methoxy-β-meta-trifluoromethylphenyl)ethylamino]ethyl benzoate hydrochloride.

**9.** d-2-[(β-methoxy-β-meta-trifluoromethylphenyl)ethylamino]ethyl benzoate hydrochloride.

**10.** Process for the preparation of the compounds of claim 1, characterised in that:

- the acid of the general formula II:

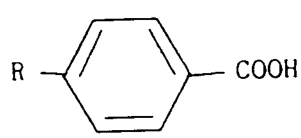

(II)

wherein R is as defined in claim 1, is converted into a salt of the general formula II':

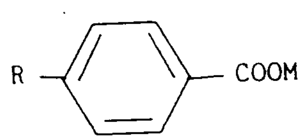

(II')

wherein R is as defined above and M is an alkali metal or an alkaline earth metal;

- the latter is reacted with a halogenated compound of the general formula III:

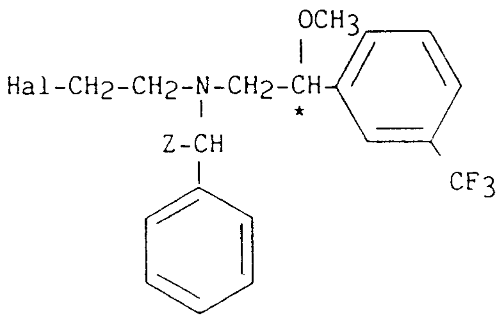

(III)

wherein
- Z represents a hydrogen atom or a methyl radical, and
- Hal represents a halogen atom;
- and the compound so obtained of the general formula IV:

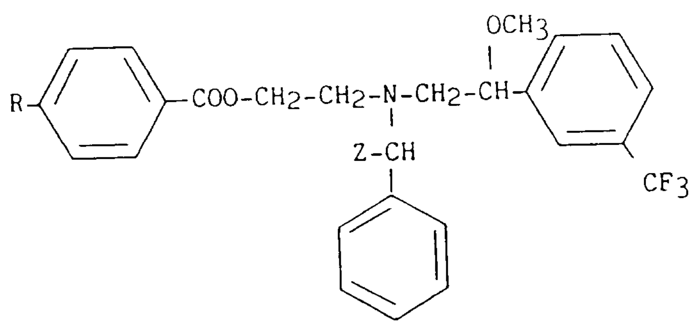

(IV),

wherein R and Z are as defined above, is debenzylated catalytically.

**11.** Pharmaceutical compositions containing as active ingredient a compound according to claims 1 to 9 with one or more appropriate pharmaceutical excipients.

**12.** Pharmaceutical compositions according to claim 11 presented in a form suitable especially for the treatment of diabetes, obesity, insulin resistance syndrome, "Syndrome X", secondary or otherwise to hyperamylinaemia involving a reduction in glucose tolerance, hyperinsulinaemia, dyslipidaemia and hypertension, and for the treatment of hypertension in insulin-resistant subjects or subjects having one or more metabolic anomalies.

**Claims for the following Contracting States : ES, GR**

**1.** Process for the preparation of ethanolamine benzoate compounds of the general formula I:

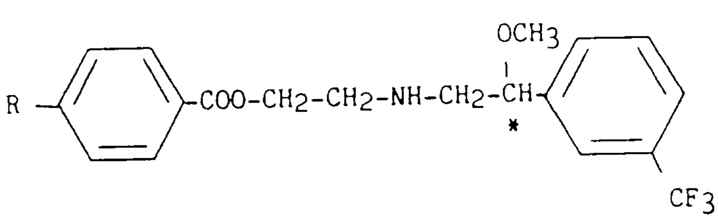

(I)

wherein:
R represents:

- a hydrogen atom or
- a group of the formula:

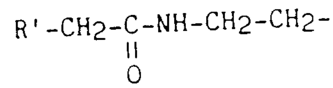

wherein R' represents:

a) either a radical of the formula:

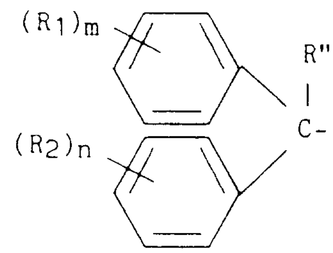

wherein:

R'' represents a hydrogen atom or an alkyl radical containing from 1 to 5 carbon atoms in a straight or branched chain,

each of $R_1$ and $R_2$, which may be identical or different, represents a hydrogen atom or an alkyl or alkoxy radical each having from 1 to 5 carbon atoms in a straight or branched chain, and

each of m and n, which may be identical or different, represents 1, 2 or 3;

b) or a fluorenyl radical of the formula:

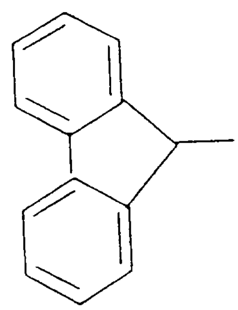

in the form of racemic compounds or enantiomers,
and their physiologically tolerable salts with appropriate acids,
characterised in that:

- the acid of the general formula II:

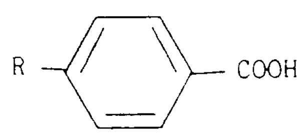

(II)

wherein R is as defined above, is converted into a salt of the general formula II':

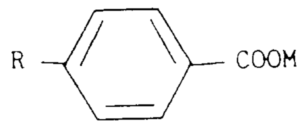

(II')

wherein R is as defined above and M is an alkali metal or an alkaline earth metal;

- the latter is reacted with a halogenated compound of the general formula III:

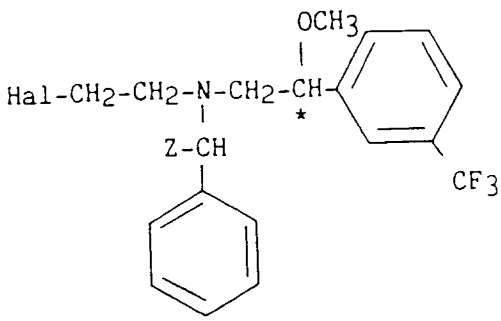

(III)

wherein

- Z represents a hydrogen atom or a methyl radical, and
- Hal represents a halogen atom;
- and the compound so obtained of the general formula IV:

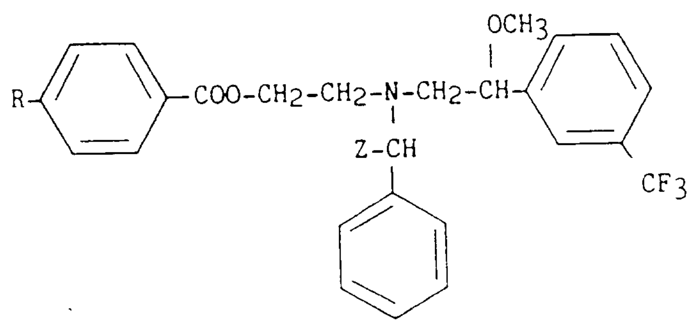

(IV),

wherein R and Z are as defined above, is debenzylated catalytically,

and, if desired, the compounds of formula I so obtained are treated with physiologically tolerable acids to give the corresponding acid addition salts.

**Patentansprüche**

**Patentansprüche für folgende Vertragsstaaten : AT, BE, CH, DE, DK, FR, GB, IT, LI, LU, NL, PT, SE**

1. Benzoesäureethanolaminester-Verbindungen der allgemeinen Formel I:

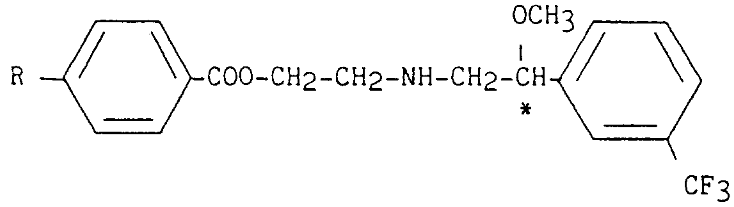

(I)

in der:

R:

- ein Wasserstoffatom oder
- eine Gruppe der Formel:

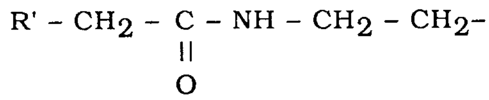

In der R':

a) entweder eine Gruppe der Formel:

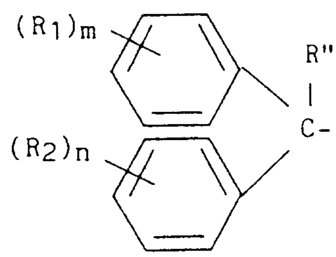

in der:

R'' ein Wasserstoffatom oder eine Alkylgruppe mit 1 bis 5 Kohlenstoffatomen In gerader oder verzweigter Kette,

$R_1$ und $R_2$, die gleichartig oder verschieden sind, jeweils ein Wasserstoffatom oder eine Alkyl- oder Alkoxy-gruppe mit jeweils 1 bis 5 Kohlenstoffatomen in gerader oder verzweigter Kette und m und n, die gleichartig oder verschieden sein können, jeweils 1, 2 oder 3 darstellen;

b) oder eine Fluorenylgruppe der Formel:

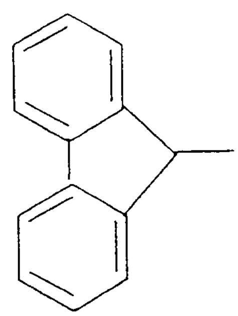

bedeuten, in Form der racemischen Verbindung oder der Enantiomeren.

**2.** Physiologisch vertragliche Salze der Verbindungen nach Anspruch 1 mit geeigneten Sauren.

**3.** l-p-[2-(α-Fluoren-9-yl-acetylamino)-ethyl]-benzoesäure-2-[[β-methoxy-β-m-trifluormethylphenyl)-ethylamino]-ethylester-hydrochlorid.

**4.** d-p-[2-[α-Fluoren-9-yl-acetylamino)ethyl]-benzoesäure-2-[(β-methoxy-β-m-trifluormethylphenyl)-ethylamino]-ethylester-hydrochlorid.

**5.** dl-p-[2-(α-Fluoren-9-yl-acetylamino)-ethyl]-benzoesaure-2-[(β-methoxy-β-m-trifluormethylphenyl)-ethylamino]ethylester-hydrochlorid.

**6.** l-p-[2-(Benzhydrylacetylamino)-ethyl]-benzoesäure-2-[(β-methoxy-β-m-trifluormethylphenyl)-ethylamino]-ethylester-hydrochlorid.

**7.** dl-2-[[β-methoxy-β-m-trifluormethylphenyl)-ethylamino]-ethylester-hydrochlorid.

**8.** l-2-[(β-methoxy-β-m-trifluormethylphenyl)-ethylamino]-ethylester-hydrochlorid.

**9.** d-2-[(β-methoxy-β-m-trifluormethylphenyl)-ethylamino]-ethylester-hydrochlorid.

**10.** Verfahren zur Herstellung der Verbindungen nach Anspruch 1, **dadurch gekennzeichnet**, daß man:

- die Saure der allgemeinen Formel II:

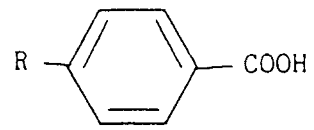

(II)

in der R die in Anspruch 1 angegebenen Bedeutungen besitzt, in ein Salz der allgemeinen Formel II':

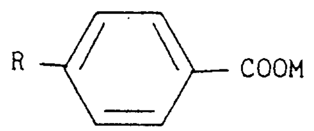

(II')

in der R die oben angegebenen Bedeutungen besitzt und M ein Alkalimetall oder ein Erdalkalimetall darstellt, umwandelt:

- dieses mit einer Halogenverbindung der allgemeinen Formel III:

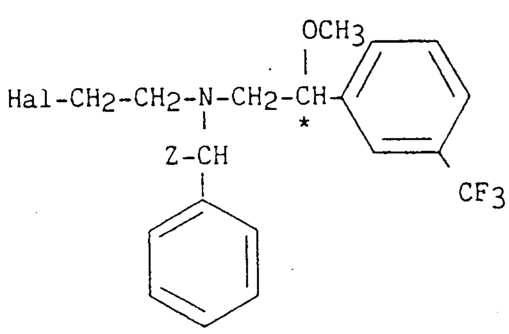

(III)

in der

- Z ein Wasserstoffatom oder eine Methylgruppe und
- Hal ein Halogenatom darstellen,

umsetzt; und

- die in dieser Weise erhaltene Verbindung der allgemeinen Formel IV:

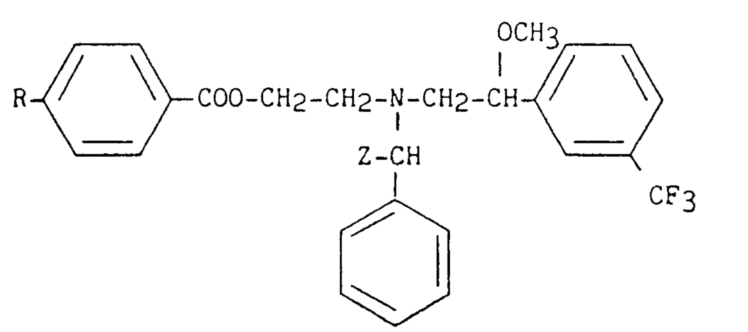

(IV)

in der R und Z die oben angegebenen Bedeutungen besitzen, katalytisch debenzyliert.

**11.** Pharmazeutische Zubereitungen enthaltend als Wirkstoff eine Verbindung nach einem der Ansprüche 1 bis 9 zusammen mit einem oder mehreren pharmazeutisch geeigneten Trägermaterialien.

**12.** Pharmazeutische Zubereitungen nach Anspruch 11 in einer Form, die insbesondere bei der Behandlung des Diabetes, der Fettsucht, des Insulinresistenzsyndroms, des "Syndroms X", das gegebenenfalls eine Folge einer Hyperamylinämie ist, die mit einer Verminderung der Verträglichkeit für Glucose, einer Hyperinsulinämie, einer Dyslipidämie und der Hypertension verknüpft ist, und zur Behandlung der Hypertension bei insulinresistenten Patienten oder solchen, die eine oder mehrere Stoffwechselanomalien aufweisen, geeignet ist.

**Patentansprüch für folgende Vertragsstaaten : ES, GR**

**1.** Verfahren zur Herstellung von Benzoesäureethanolaminester-Verbindungen der allgemeinen Formel I:

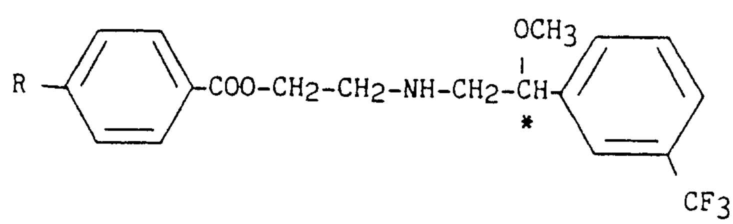

(I)

in der:

R:

- ein Wasserstoffatom oder
- eine Gruppe der Formel:

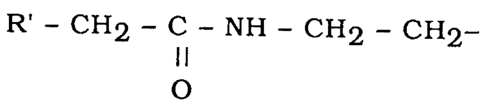

in der R':

a) entweder eine Gruppe der Formel:

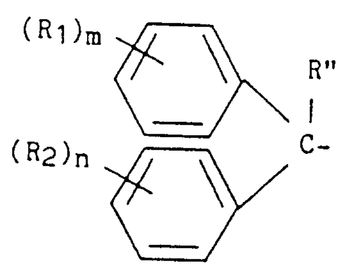

in der:

R" ein Wasserstoffatom oder eine Alkylgruppe mit 1 bis 5 Kohlenstoffatomen in gerader oder verzweigter Kette,

$R_1$ und $R_2$, die gleichartig oder verschieden sind, jeweils ein Wasserstoffatom oder eine Alkyl- oder Alkoxygruppe mit jeweils 1 bis 5 Kohlenstoffatomen in gerader oder verzweigter Kette und m und n, die gleichartig oder verschieden sein können, jeweils 1, 2 oder 3 darstellen;

b) oder eine Fluorenylgruppe der Formel:

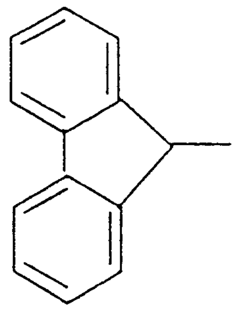

bedeuten, in Form der racemischen Verbindung oder der Enantiomeren,
und von deren physiologisch verträglichen Salzen mit geeigneten Säuren, **dadurch gekennzeichnet**, daß man:

- die Säure der allgemeinen Formel II:

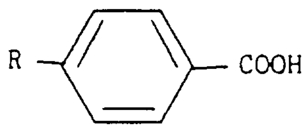

(II)

in der R die obenangegebenen Bedeutungen besitzt, in ein Salz der allgemeinen Formel II':

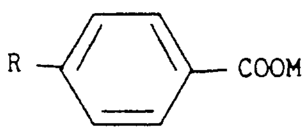

(II')

in der R die oben angegebenen Bedeutungen besitzt und M ein Alkalimetall oder ein Erdalkallmetall darstellt, umwandelt:

- dieses mit einer Halogenverbindung der allgemeinen Formel III:

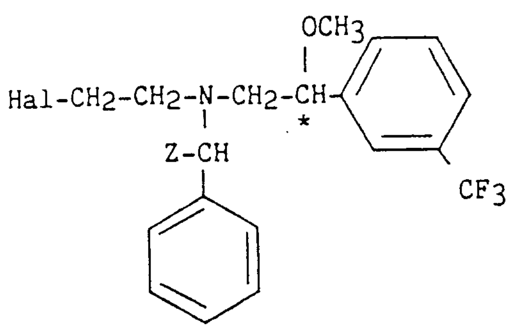

(III)

in der

- Z ein Wasserstoffatom oder eine Methylgruppe und
- Hal ein Halogenatom darstellen,

umsetzt; und

- die in dieser Weise erhaltene Verbindung der allgemeinen Formel IV:

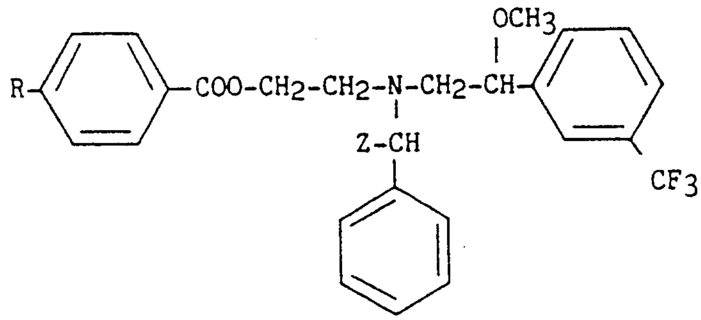

(IV)

in der R und Z die oben angegebenen Bedeutungen besitzen, katalytisch debenzyliert, und gewünschtenfalls die in diese Weise erhaltenen Verbindungen der Formel I mit physiologisch verträglichen Säuren behandelt zur Bildung der entsprechenden Säureadditionssalze.